# EUROPEAN PATENT APPLICATION

(11) **EP 4 020 412 A1**
(43) Date of publication of application: **29.06.2022**
(21) Application number: 21215531.1
(22) Date of filing: 17.12.2021
(51) Int. Cl.: G07D 11/00, G07D 11/24, G07D 11/25

(54) **BANKNOTE HANDLING APPARATUS AND BANKNOTE HANDLING METHOD**

(30) Priority: 28.12.2020 JP 2020218930
(71) Applicant: GLORY LTD., Himeji-shi Hyogo 670-8567 (JP)
(72) Inventor: MOURI, Hiroya, Hyogo 670-8567 (JP); ITO, Osamu, Hyogo 670-8567 (JP); KOBAYASHI, Kiyoaki, Hyogo 670-8567 (JP)
(74) Representative: SSM Sandmair

(57) **Abstract**

In order to efficiently perform banknote handling and banknote disinfection, a banknote handling apparatus includes: an inlet that receives a banknote from outside the apparatus; a transport unit that transports the banknotes from the inlet into the apparatus; a recognition unit that performs banknote recognition of the banknote; a disinfection unit that performs banknote disinfection of the banknote; an outlet that holds the banknotes; and a control unit that controls these units to perform a banknote handling process in which the banknote passes the recognition unit and the disinfection unit. The banknote handling process is performed in a mode selected from among a first mode of performing the banknote recognition and the banknote disinfection, a second mode of performing the banknote disinfection without performing the banknote recognition, and a third mode of performing the banknote recognition without performing the banknote disinfection.

## Description

### BACKGROUND OF THE INVENTION

### Field of the Invention

The present disclosure relates to a banknote handling apparatus and a banknote handling method for handling banknotes.

### Description of the Background Art

In recent years, highly infectious pathogens such as bacteria and viruses have been found. Therefore, people may have concern about a possibility of attaching a pathogen to banknotes and a risk of being infected with the pathogen through the banknotes. If a highly infectious pathogen may attach to banknotes dispensed from a banknote handling apparatus, the user cannot use the banknote handling apparatus safely. In order to avoid such a situation, Japanese Patent No. 2993690 and U.S. Patent No. 5374814 each disclose an apparatus for disinfecting banknotes taken into the apparatus. In this apparatus, a banknote being transported along a transport path is irradiated with an ultraviolet ray to disinfect the banknote.

### SUMMARY OF THE INVENTION

In the above conventional art, it is sometimes difficult to efficiently perform both banknote disinfection and banknote handling such as depositing of banknotes. For example, if the transport speed of banknotes is reduced to disinfect the banknotes, a time required for handling the banknote increases.

The present disclosure is made in view of the conventional art including the above problem. The present disclosure addresses the problem, as discussed herein, with a banknote handling apparatus and a banknote handling method capable of efficiently performing banknote handling and banknote disinfection.

A banknote handling apparatus according to one aspect of the present disclosure includes: a transport unit configured to transport a banknote; a recognition unit configured to recognize the banknote being transported by the transport unit; a disinfection unit configured to disinfect the banknote being transported by the transport unit; and a control unit configured to control operations of the transport unit, the recognition unit, and the disinfection unit to perform a banknote handling process in which the banknotes being transported by the transport unit pass the recognition unit and the disinfection unit.

The control unit controls other units to perform the banknote handling process in a mode selected from among a plurality of modes. For example, the plurality of modes are defined according to whether or not to perform banknote recognition to recognize the banknote by the recognition unit, and whether or not to perform banknote disinfection to disinfect the banknote by the disinfection unit. Furthermore, the plurality of modes may be defined in consideration of elements other than the banknote recognition and the banknote disinfection. A mode in which the banknote recognition is not performed may include: a mode in which the recognition unit does not operate for the banknote recognition; and a mode in which the recognition unit operates for the banknote recognition but a recognition result is not used for handling the banknote.

The control unit may control other units to perform the banknote handling process in a mode selected from a first mode of performing the banknote recognition by the recognition unit and the banknote disinfection by the disinfection unit, and a second mode of performing the banknote disinfection by the disinfection unit without performing the banknote recognition by the recognition unit. In the banknote handling process in the second mode, the banknote is not recognized even when the banknote passes the recognition unit. The banknote handling process may be performed in a mode selected from among a plurality of modes including the first and second modes, and a third mode of performing the banknote recognition by the recognition unit without performing the banknote disinfection by the disinfection unit. In the banknote handling process in the third mode, the banknote is not disinfected even when the banknote passes the disinfection unit. Moreover, the banknote handling process may be performed in a mode selected from among a plurality of modes including the first to third modes, and a fourth mode of performing neither the banknote disinfection by the disinfection unit nor the banknote recognition by the recognition unit. In the banknote handling process in the fourth mode, the banknote is neither recognized nor disinfected even when the banknote passes the recognition unit and the disinfection unit.

The banknote handling apparatus may further include an inlet configured to receive banknotes from outside the banknote handling apparatus. The transport unit transports the banknote fed out from the inlet into the apparatus. The control unit controls the operation of the inlet. The banknote handling apparatus may further include an outlet configured to hold the banknote such that the banknote can be taken out from the apparatus. The outlet receives the banknote from the transport unit. The control unit controls the operation of the outlet.

The control unit may control the transport unit such that a banknote transport speed during the banknote handling process in the mode of performing the banknote disinfection is different from a banknote transport speed during the banknote handling process in the mode of not performing the banknote disinfection. For example, the control unit may control the transport unit such that the banknote transport speed in the mode of not performing the banknote disinfection is higher than the banknote transport speed in the mode of performing the banknote disinfection. Specifically, the control unit may control the transport unit such that the banknote transport speed during the banknote handling process in the first mode is different from the banknote transport speed during the banknote handling process in the third mode. The control unit may control the transport unit such that the banknote transport speed during the banknote handling process in the second mode is different from the banknote transport speed during the banknote handling process in the third mode.

The same banknote may be disinfected by the disinfection unit a plural number of times during one banknote handling process. The control unit may consecutively perform the banknote handling process a plural number of times in the mode of performing the banknote disinfection so as to disinfect the same banknote a plural number of times. Specifically, the control unit may repeatedly perform the banknote handling process in the first mode or the second mode to disinfect the same banknote a plural number of times. The control unit may consecutively perform two or more times the banknote handling process in the mode of performing the banknote disinfection, and one or more times the banknote handling process in the mode of not performing the banknote disinfection.

The control unit may change the banknote transport speed in the banknote handling process in the mode of performing the banknote disinfection, according to the type of the banknote handling process. The types of the banknote handling process may include at least two types of processes among a depositing process, a dispensing process, a counting process, an inventory confirming process, a replenishment process, and a collection process. For example, the control unit may control the transport unit such that the banknote transport speed in the dispensing process in the mode of performing the banknote disinfection is higher than the banknote transport speed in the depositing process in the mode of performing the banknote disinfection. The types of the banknote handling processes may be classified according to the number of times the banknote disinfection is performed for one banknote. For example, the control unit may control the transport unit such that the banknote transport speed when performing the banknote handling process in which the banknote disinfection is performed a plural number of times for one banknote is higher than the banknote transport speed when performing the banknote handling process in which the banknote disinfection is performed only once for one banknote.

In the case where the banknote disinfection is performed a plural number of times for one banknote during the banknote handling process, the control unit may control the transport unit such that the banknote transport speed when the first banknote disinfection is performed is different from the banknote transport speed when the second banknote disinfection is performed. This type of banknote handling process includes a process of causing the banknote to reciprocate on the transport unit. For example, in this type of banknote handling process, the banknote passes the recognition unit in both the forward path and the return path, and the banknote transport speed in the forward path is different from the banknote transport speed in the return path.

In the case where the disinfection unit disinfects the same banknote a plural number of times, the control unit may display, on the display, information indicating the number of times of the banknote disinfection to be performed and/or the counted number of times of the banknote disinfection that has been performed.

The control unit may invalidate a part of recognition functions of the recognition unit when performing the banknote handling process in the mode of performing the banknote recognition. In the case where the recognition unit has recognition functions of recognizing the number of banknotes, denomination, authenticity, and fitness of each banknote, the banknote handling process is performed in the first mode or the third mode, and the control unit may control the recognition unit not to perform at least one of the recognition functions regarding the number of banknotes, denomination, authenticity, and fitness of each banknote. For example, the control unit may control the recognition unit to recognize only the number of banknotes without recognizing denomination, authenticity, and fitness of each banknote.

The banknote handling process may be a process in which the inlet feeds out the banknotes received from outside the apparatus into the apparatus, the transport unit transports the banknotes fed out from the inlet, and the outlet receives the banknotes transported by the transport unit.

The banknote handling apparatus may further include: a storage unit configured to store the banknotes received from the transport unit, and feed out the stored banknotes to the transport unit; and a temporary storage unit configured to temporarily store the banknotes received from the transport unit, and feed out the temporarily stored banknotes to the transport unit. The banknote handling apparatus may perform the banknote handling process in which the transport unit transports the banknotes fed out from the storage unit, and the temporary storage unit receives the banknotes transported by the transport unit and temporarily stores the banknotes therein. The banknote handling apparatus may perform the banknote handling process in which the transport unit transports the banknotes fed out from the temporary storage unit, and the storage unit receives the banknotes transported by the transport unit and stores the banknotes therein. The banknote handling apparatus may perform the banknote handling process in which the transport unit transports the banknotes fed out from the storage unit, the temporary storage unit receives the banknotes transported by the transport unit and temporarily stores the banknotes therein, the transport unit transports the banknotes fed out from the temporary storage unit, and the storage unit receives the banknotes transported by the transport unit and stores the banknotes therein.

The recognition unit and the disinfection unit may be disposed adjacent to each other on the transport unit. In the banknote handling process according to the present disclosure, banknotes pass the recognition unit and the disinfection unit. The recognition unit and the disinfection unit may be connected to each other by a transport path, which has no divergent path and constitutes a part of the transport unit. In this case, during the banknote handling process according to the present disclosure, a banknote that passes the recognition unit also passes the disinfection unit, and a banknote that passes the disinfection unit also passes the recognition unit.

A banknote handling method according to the present disclosure is a method performed by a banknote handling apparatus, and the method includes performing a step selected from among the steps of: recognizing, by a recognition unit, banknotes received from outside the banknote handling apparatus, and disinfecting the banknotes by a disinfection unit; disinfecting, by the disinfection unit, the banknotes received from outside the banknote handling apparatus, without recognizing the banknotes by the recognition unit; and recognizing, by the recognition unit, the banknotes received from outside the banknote handling apparatus, without disinfecting the banknotes by the disinfection unit.

According to the present disclosure, banknote handling and banknote disinfection can be efficiently performed by using the banknote handling apparatus.

### BRIEF DESCRIPTION OF THE DRAWINGS

FIG. 1 illustrates a banknote handling apparatus according to the present embodiment;
FIG. 2 shows an example of a banknote handling apparatus;
FIG. 3 illustrates a depositing process not using a temporary storage unit;
FIGS. 4A and 4B illustrate a depositing process using the temporary storage unit;
FIG. 5 illustrates a dispensing process not using the temporary storage unit;
FIGS. 6A and 6B illustrate a dispensing process using the temporary storage unit;
FIGS. 7A and 7B illustrate an inventory confirming process;
FIGS. 8A and 8B illustrate a method of repeating banknote disinfection;
FIG. 9 illustrates a counting process not using the temporary storage unit;
FIGS. 10A and 10B illustrate a counting process using the temporary storage unit;
FIGS. 11A and 11B show an example of screens displayed on a display;
FIGS. 12A to 12C illustrate a money exchange process;
FIG. 13 shows an example of a banknote handling apparatus to/from which a cassette is mountable/dismountable;
FIG. 14 shows another example of the banknote handling apparatus to/from which the cassette is mountable/dismountable shown in FIG. 13;
FIGS. 15A and 15B illustrate a depositing process and a dispensing process of a front apparatus;
FIG. 16 illustrates a depositing process, for banknotes in a cassette, performed without using the temporary storage unit;
FIGS. 17A and 17B illustrate a depositing process, for banknotes in the cassette, performed using the temporary storage unit;
FIG. 18 illustrates a dispensing process, to the cassette, performed without using a temporary storage unit;
FIGS. 19A and 19B illustrate a dispensing process, to the cassette, performed by using the temporary storage unit; and
FIGS. 20A and 20B illustrate an example of a banknote handling apparatus that does not store banknotes in the storing unit inside he banknote handling apparatus.

### DESCRIPTION OF THE PREFERRED EMBODIMENTS

Hereinafter, an embodiment of a banknote handling apparatus and a banknote handling method according to the present disclosure will be described with reference to the accompanying drawings. The banknote handling apparatus according to the present embodiment includes one or a plurality of disinfection units to disinfect banknotes. In this embodiment, the "disinfection" includes a process for killing pathogens such as bacteria and viruses that may attach to banknotes. The "disinfection" is not limited to a process of completely killing the pathogens, and may be a process of decreasing the number of pathogens or weakening the pathogens to an extent that does not cause any problem such as infection in using the banknotes.

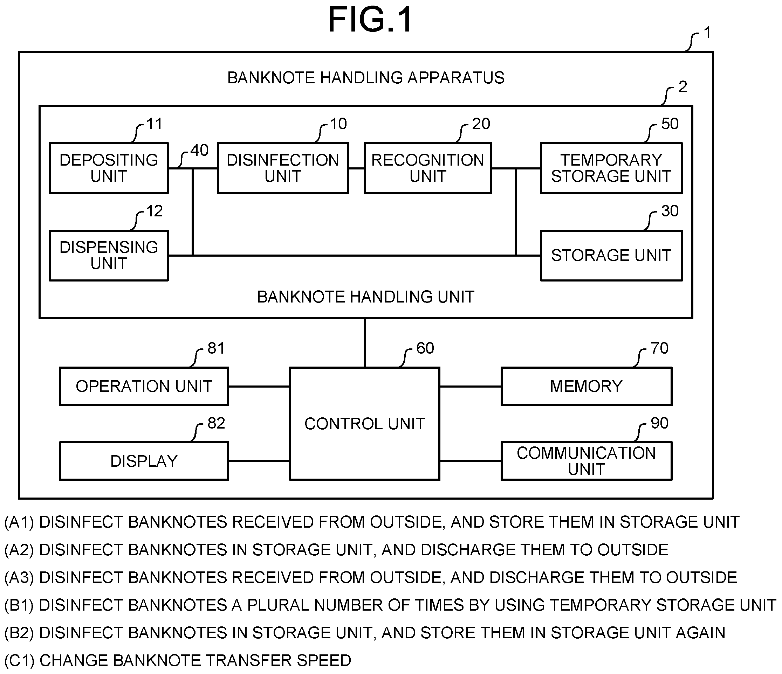

FIG. 1 illustrates an example of a banknote handling apparatus 1 according to the present embodiment. As shown in FIG. 1, the banknote handling apparatus 1 includes a banknote handling unit 2 for handling banknotes, a memory 70 that stores information related to banknote handling processes, and a control unit 60 that controls the banknote handling unit 2 while using the memory 70.

The memory 70 is a nonvolatile storage device. The memory 70 stores information necessary for the banknote handling apparatus 1 to perform banknote handling processes. The control unit 60 controls the banknote handling unit 2 while using the information stored in the memory 70, to realize functions and operations of the banknote handling apparatus 1 described in the present embodiment.

As shown in FIG. 1, the banknote handling apparatus 1 may include an operation unit 81, a display 82, and a communication unit 90. The operation unit 81 is used for inputting information related to banknote handling processes. The control unit 60 can control the banknote handling unit 2 to perform banknote handling processes, based on information inputted through the operation unit 81. The display 82 is used for displaying information related to banknote handling processes. The control unit 60 can notify an operator of the banknote handling apparatus 1, of the information related to banknote handling processes through the display 82. The communication unit 90 is used for exchanging information related to banknote handling processes with an external device. The control unit 60 can control the banknote handling unit 2 to perform banknote handling processes, based on information that the communication unit 90 has received from the external device. Alternatively, the banknote handling apparatus 1 may be independently used without including the communication unit 90. The operation unit 81 and the display 82 may be integrated with each other. At least one of the operation unit 81 and the display 82 may be disposed independently of the banknote handling apparatus 1. For example, an operation terminal including the operation unit 81 and the display 82 may be connected to the banknote handling apparatus 1, and information related to banknote handling processes may be inputted/outputted through the operation terminal.

The banknote handling unit 2 is configured to perform banknote handling processes. The banknote handling unit 2 includes a depositing unit 11, a dispensing unit 12, a disinfection unit 10, a recognition unit 20, and a transport unit 40. The banknote handling unit 2 may further include a storage unit 30 and a temporary storage unit 50.

The transport unit 40 is configured to transport banknotes between the depositing unit 11, the dispensing unit 12, the disinfection unit 10, the recognition unit 20, the storage unit 30, and the temporary storage unit 50. The configuration of the transport unit 40 indicated by solid lines in FIG. 1 is an example and is not limited thereto. The manner of connecting the respective units by the transport unit 40 can be changed from that shown in FIG. 1 as long as each banknote to be handled in the banknote handling unit 2 can be disinfected by using the disinfection unit 10 as described in the present embodiment.

The depositing unit 11 functions as an inlet. The depositing unit 11 is configured to receive banknotes from the outside of the banknote handling apparatus 1. The depositing unit 11 feeds out the banknotes therefrom to the transport unit 40 inside the apparatus. The depositing unit 11 receives a plurality of banknotes to be handled in one banknote handling process, and feeds out these banknotes one by one. The depositing unit 11 is provided with a sensor capable of detecting whether or not all the banknotes in the depositing unit 11 have been fed out to the transport unit 40.

The dispensing unit 12 functions as an outlet. The dispensing unit 12 is configured to hold banknotes received from the transport unit 40 such that the banknotes can be taken out of the money handling apparatus 1. The dispensing unit 12 is used for discharging, from the money handling apparatus 1, the banknotes having been transported one by one by the transport unit 40. The dispensing unit 12 can stack a plurality of banknotes therein. The depositing unit 11 and the dispensing unit 12 may not necessarily be disposed independently from each other as shown in FIG. 1, and one component may serve both as the depositing unit 11 and the dispensing unit 12.

The recognition unit 20 is configured to recognize the banknotes being transported by the transport unit 40. For example, the recognition unit 20 recognizes denomination of each banknote. The recognition unit 20 may further recognize authenticity and fitness of each banknote. The recognition unit 20 may count the number of banknotes and the monetary amount of banknotes. For example, when a plurality of banknotes are handled in one banknote handling process, the recognition unit 20 counts at least one of the number of banknotes for each denomination, the amount of banknotes for each denomination, the total number of banknotes, and the total amount of banknotes.

The storage unit 30 is configured to store the banknotes transported by the transport unit 40. The storage unit 30 is configured to feed out the stored banknotes to the transport unit 40. The number of storage units 30 included in the banknote handling apparatus 1 is not particularly limited. The storage unit 30 may be disposed inside the apparatus so as not to be visible from the outside. For example, the banknote handling apparatus 1 may include a metallic safe, in which the storage unit 30 may be disposed. The storage unit 30 may not necessarily perform both storage and feeding-out of banknotes. The storage unit 30 may perform only feeding-out of banknotes to the transport unit 40 without performing storage of banknotes received from the transport unit 40. In this case, the banknote handling apparatus 1 may perform a process in which storage of banknotes into the storage unit 30 is not necessary, among processes described in this embodiment. The storage unit 30 may perform only storage of banknotes received from the transport unit 40 without performing feeding-out of banknotes to the transport unit 40. In this case, the banknote handling apparatus 1 may perform a process in which feeding-out of banknotes from the storage unit 30 is not necessary, among the processes described in this embodiment.

The disinfection unit 10 is configured to disinfect each banknote being transported by the transport unit 40. The disinfection unit 10 includes a light source for disinfecting the banknotes. For example, the light source emits an ultraviolet ray having a disinfection effect. For example, two light sources are disposed opposed to each other with the transport unit 40 interposed therebetween, so that both sides of one banknote being transported by the transport unit 40 are irradiated with ultraviolet rays. The disinfection unit 10 can irradiate, with the ultraviolet rays, the entire face side and the entire back side of the banknote being transported by the transport unit 40. For example, each light source of the disinfection unit 10 has a shape elongated in a direction orthogonal to the transport direction along which banknotes are transported by the transport unit 40. When the banknote being transported by the transport unit 40 passes an irradiation area of the ultraviolet rays from the light sources, the entirety of the banknote is irradiated with the ultraviolet rays. For example, each light source is disposed at a position where the distance from the light source to the face side or the back side of the banknote is equal to or smaller than 1.2 mm. Each light source may be disposed such that the distance may be equal to or larger than 0.3 mm for transporting banknotes smoothly and safely.

For example, the disinfection unit 10 irradiates the banknote with an ultraviolet ray in a wavelength range called UVC that has a high disinfection ability. The disinfection unit 10 may irradiate the banknote with an ultraviolet ray at a predetermined wavelength or in a predetermined wavelength range, in the UVC range (280 nm and shorter).

It is preferable that the disinfection unit 10 emits an ultraviolet ray having a high disinfection ability. However, in consideration of an influence to a human body, when there is a risk that the ultraviolet ray emitted from the disinfection unit 10 leaks to outside the banknote handling apparatus 1, it is preferable that the disinfection unit 10 emits an ultraviolet ray that has a disinfection ability and is safe for the human body. For example, when there is no risk of leakage of an ultraviolet ray from the apparatus, the disinfection unit 10 that emits an ultraviolet ray in a wavelength range of 250-260 nm is disposed inside the apparatus. Meanwhile, when there is a risk of leakage of an ultraviolet ray from the apparatus, the disinfection unit 10 that emits an ultraviolet ray having a wavelength of 222 nm is disposed inside the apparatus.

Specifically, for example, the disinfection unit 10 that emits an ultraviolet ray of 222 nm is used in the banknote handling apparatus 1 having a risk that the ultraviolet ray emitted from the disinfection unit 10 leaks to outside the apparatus through an inlet portion extending from the depositing unit 11 to the transport unit 40, or an outlet portion extending from the transport unit 40 to the dispensing unit 12. For another example, the disinfection unit 10 that emits an ultraviolet ray of 222 nm is used in the banknote handling apparatus 1 in which the storage unit 30 has an opening for taking out banknotes from the apparatus and the opening may cause a risk of leakage of the ultraviolet ray through an outlet portion extending from the transport unit 40 to the storage unit 30.

In the example shown in FIG. 1, the disinfection unit 10 is disposed adjacent to the recognition unit 20. The disinfection unit 10 is connected to the recognition unit 20 by a transport path that constitutes a part of the transport unit 40. In the example shown in FIG. 1, the transport path connecting the disinfection unit 10 and the recognition unit 20 has no divergent path and therefore, a banknote that passed the disinfection unit 10 passes the recognition unit 20, and a banknote that passed the recognition unit 20 passes the disinfection unit 10. That is, the banknote handling apparatus 1 is configured to perform a banknote handling process in which each banknote can pass both the disinfection unit 10 and the recognition unit 20. However, the position of the disinfection unit 10 and the number of the disinfection units 10 shown in FIG. 1 are an example, and are not particularly limited. The positions of the disinfection unit 10 and the recognition unit 20 shown in FIG. 1 may be interchanged. One disinfection unit 10 may be disposed at a position different from that shown in FIG. 1, or two or more disinfection units 10 may be disposed in the banknote handling apparatus 1. The disinfection unit 10 may not necessarily be independently disposed and may be integrated with another unit. For example, the disinfection unit 10 may be included in the recognition unit 20.

The banknote handling apparatus 1 can perform a banknote handling process in an operation mode of disinfecting banknotes by the disinfection unit 10 or an operation mode of not disinfecting banknotes. In a banknote handling process referred to as "disinfection mode", banknotes are disinfected by the disinfection unit 10. The banknote handling apparatus 1 can perform a banknote handling process in an operation mode of recognizing banknotes by the recognition unit 20 or an operation mode of not recognizing banknotes. In a banknote handling process referred to as "recognition mode", banknotes are recognized by the recognition unit 20.

The banknote handling apparatus 1 can perform a banknote handling process, in which banknotes are received in the depositing unit 11 from outside the money handling apparatus 1 and stored in the storage unit 30, in the disinfection mode (A1). For example, in a depositing process for depositing banknotes into the banknote handling apparatus 1, the banknotes fed out from the depositing unit 11 into the apparatus 1 are transported by the transport unit 40 to the disinfection unit 10 one by one. The disinfection unit 10 irradiates each banknote being transported by the transport unit 40 with the ultraviolet ray to disinfect the banknote. The transport unit 40 transports each banknote from the disinfection unit 10 to the recognition unit 20. The recognition unit 20 recognizes each banknote being transported by the transport unit 40. The transport unit 40 transports each banknote from the recognition unit 20 to the storage unit 30. The storage unit 30 stores each banknote therein. For example, information on the banknotes obtained through the recognition by the recognition unit 20 is used for managing the banknotes stored in the storage unit 30. The information on the deposited banknotes may be displayed on the display 82, or may be transmitted by the communication unit 90 to the external device.

The banknote handling apparatus 1 can perform a banknote handling process, in which banknotes are fed out from the storage unit 30 and discharged from the dispensing unit 12 to outside the money handling apparatus 1, in the disinfection mode (A2). For example, in a dispensing process for dispensing banknotes stored in the storage unit 30 from the banknote handling apparatus 1, the banknotes fed out from the storage unit 30 to the transport unit 40 are transported to the recognition unit 20 one by one. The recognition unit 20 recognizes each banknote being transported by the transport unit 40. The transport unit 40 transports each banknote from the recognition unit 20 to the disinfection unit 10. The disinfection unit 10 irradiates each banknote being transported by the transport unit 40 with the ultraviolet ray to disinfect the banknote. The transport unit 40 transports each banknote from the disinfection unit 10 to the dispensing unit 12. The dispensing unit 12 discharges each banknote to outside the money handling apparatus 1. For example, information on the banknotes obtained through the recognition by the recognition unit 20 is used for managing banknotes being stored in the storage unit 30. The information on the dispensed banknotes may be displayed on the display 82, or may be transmitted by the communication unit 90 to the external device.

The dispensing process may be performed in an operation mode in which the recognition unit 20 does not recognize banknotes. For example, in the case where the control unit 60 manages, in the memory 70, information on the banknotes being stored in the storage unit 30 and therefore can specify the denomination of each banknote fed out from the storage unit 30, banknote recognition by the recognition unit 20 may be omitted. An operator can select whether or not the recognition unit 20 recognizes each banknote to be dispensed, by changing the setting on the dispensing process.

The banknote handling apparatus 1 can perform a banknote handling process in the disinfection mode in which banknotes received in the depositing unit 11 from outside the apparatus 1 are taken into the apparatus 1, and discharged from the dispensing unit 12 to outside the apparatus 1 (A3). That is, the transport unit 40 continues transporting a banknote received in the depositing unit 11, the banknote is disinfected by the disinfection unit 10 while being transported from the depositing unit 11 to the dispensing unit 12, and the disinfected banknote is discharged from the dispensing unit 12 without storing in the storage unit 30.

For example, the banknote handling apparatus 1 can perform a counting process, in which banknotes are received in the depositing unit 11, taken into the apparatus 1, counted and returned from the dispensing unit 12, in an operation mode of performing both banknote recognition and banknote disinfection. In the counting process in this operation mode, the depositing unit 11 feeds the banknotes to the transport unit 40 one by one, and the transport unit 40 transports each banknote to the disinfection unit 10. The disinfection unit 10 irradiates each banknote being transported by the transport unit 40 with the ultraviolet ray to disinfect the banknote. The transport unit 40 transports each banknote from the disinfection unit 10 to the recognition unit 20. The recognition unit 20 recognizes each banknote being transported by the transport unit 40. The transport unit 40 transports each banknote from the recognition unit 20 to the dispensing unit 12. Each banknote is discharged from the dispensing unit 12 to outside the money handling apparatus 1. For example, a counting result obtained through the recognition by the recognition unit 20 is displayed on the display 82. The counting result may be transmitted by the communication unit 90 to the external device.

For example, the banknote handling apparatus 1 can perform a disinfection process in which banknotes are received in the depositing unit 11, taken into the apparatus 1, disinfected and returned from the dispensing unit 12. The disinfection process is performed in an operation mode of performing the banknote disinfection without performing the banknote recognition. In the disinfection process, the depositing unit 11 feeds the banknotes to the transport unit 40 one by one, and the transport unit 40 transports each banknote to the disinfection unit 10. The disinfection unit 10 irradiates each banknote being transported by the transport unit 40 with the ultraviolet ray to disinfect the banknote. The transport unit 40 transports each banknote from the disinfection unit 10 to the dispensing unit 12 via the recognition unit 20. The dispensing unit 12 discharges each banknote to outside the apparatus 1. In the disinfection process, the banknote disinfected by the disinfection unit 10 passes the recognition unit 20 but is not subjected to the recognition process, and is discharged from the dispensing unit 12.

The banknote handling apparatus 1 can disinfect one banknote a plural number of times during one banknote handling process, by using the temporary storage unit 50 (B1). For example, in the depositing process in the operation mode of performing the banknote disinfection and the banknote recognition, the banknotes fed out from the depositing unit 11 one by one to the transport unit 40 are disinfected by the disinfection unit 10 and recognized by the recognition unit 20, and thereafter each banknote is temporarily stored in the temporary storage unit 50. After all the banknotes in the depositing unit 11 have been temporarily stored in the temporary storage unit 50, the temporary storage unit 50 feeds out all the temporarily stored banknotes to the transport unit 40 one by one. The fed-out banknotes are transported to the disinfection unit 10 via the recognition unit 20, and disinfected by the disinfection unit 10. After being disinfected by the disinfection unit 10, these banknotes are transported to the storage unit 30 and stored therein. As a result, each banknote is disinfected twice during one depositing process.

When the depositing process is performed in the recognition mode by using the temporary storage unit 50 as described above, the banknote recognition of each banknote by the recognition unit 20 may not necessarily be always performed on the way to the temporary storage unit 50 from the depositing unit 11 via the disinfection unit 10. The recognition unit 20 may recognize the banknote on the way to the temporary storage unit 50 from the depositing unit 11 via the disinfection unit 10, and/or on the way to the storage unit 30 from the temporary storage unit 50 via the disinfection unit 10. Even when the temporary storage unit 50 is used for the depositing process, the banknotes may be disinfected only once on the way to the temporary storage unit 50 from the depositing unit 11, or on the way to the storage unit 30 from the temporary storage unit 50.

When the dispensing process is performed in the disinfection mode, the banknote handling apparatus 1 can disinfect one banknote a plural number of times during one banknote handling process, by using the temporary storage unit 50. Specifically, the banknotes fed out from the storage unit 30 one by one to the transport unit 40 are directly transported to the disinfection unit 10 without passing the recognition unit 20, and disinfected by the disinfection unit 10. Thereafter, the banknotes are recognized by the recognition unit 20, transported to the temporary storage unit 50 and temporarily stored therein. After all the banknotes to be dispensed have been temporarily stored in the temporary storage unit 50, the temporary storage unit 50 feeds out all the temporarily stored banknotes to the transport unit 40 one by one. The fed-out banknotes are transported to the disinfection unit 10 via the recognition unit 20, disinfected by the disinfection unit 10, and discharged to outside the apparatus 1 from the dispensing unit 12. As a result, each banknote is disinfected twice during one dispensing process.

When the dispensing process is performed in the recognition mode by using the temporary storage unit 50, the banknote recognition of each banknote by the recognition unit 20 may be performed on the way to the temporary storage unit 50 from the storage unit 30 vis the disinfection unit 10, and/or on the way to the dispensing unit 12 from the temporary storage unit 50 via the disinfection unit 10. When the dispensing process is performed without recognizing banknotes, banknotes to be dispensed from the storage unit 30 are specified based on the information managed in the memory 70 and these banknotes can be disinfected in the same way as the dispensing process in the recognition mode. Even when the temporary storage unit 50 is used for the dispensing process, the banknotes may be disinfected only once on the way to the temporary storage unit 50 from the storage unit 30, or on the way to the dispensing unit 12 from the temporary storage unit 50.

When the counting process is performed in the disinfection mode, the banknote handling apparatus 1 can disinfect one banknote a plural number of times during one banknote handling process, by using the temporary storage unit 50. Specifically, the banknotes fed out from the depositing unit 11 one by one to the transport unit 40 are disinfected by the disinfection unit 10 and recognized by the recognition unit 20, and thereafter these banknotes are temporarily stored in the temporary storage unit 50. After all the banknotes in the depositing unit 11 have been temporarily stored in the temporary storage unit 50, the temporary storage unit 50 feeds out all the temporarily stored banknotes to the transport unit 40 on by one. The fed-out banknotes are transported to the disinfection unit 10 via the recognition unit 20, disinfected by the disinfection unit 10, and discharged to outside the apparatus 1 from the dispensing unit 12. As a result, each banknote is disinfected twice during one counting process.

When the counting process is performed in the recognition mode by using the temporary storage unit 50, the banknote recognition of each banknote by the recognition unit 20 may not necessarily be always performed on the way to the temporary storage unit 50 from the depositing unit 11 via the disinfection unit 10. The recognition unit 20 may recognize the banknote on the way to the temporary storage unit 50 from the depositing unit 11 via the disinfection unit 10, and/or on the way to the dispensing unit 12 from the temporary storage unit 50 via the disinfection unit 10. Even when the temporary storage unit 50 is used for the counting process, the banknotes may be disinfected only once on the way to the temporary storage unit 50 from the depositing unit 11, or on the way to the dispensing unit 12 from the temporary storage unit 50.

In the disinfection process, the banknote handling apparatus 1 can disinfect one banknote a plural number of times during one banknote handling process, by using the temporary storage unit 50. Specifically, the banknotes fed out from the depositing unit 11 one by one to the transport unit 40 are disinfected by the disinfection unit 10, transported to the temporary storage unit 50 via the recognition unit 20 and temporarily stored in the temporary storage unit 50. After all the banknotes in the depositing unit 11 have been temporarily stored in the temporary storage unit 50, the temporary storage unit 50 feeds out all the temporarily stored banknotes to the transport unit 40 one by one. The fed-out banknotes are transported to the disinfection unit 10 via the recognition unit 20, disinfected by the disinfection unit 10, and discharged to outside the apparatus 1 from the dispensing unit 12. As a result, each banknote is disinfected twice during one disinfection process. Even when the temporary storage unit 50 is used for the disinfection process, the banknotes may be disinfected only once on the way to the temporary storage unit 50 from the depositing unit 11, or on the way to the dispensing unit 12 from the temporary storage unit 50.

The banknote handling apparatus 1 can disinfect banknotes stored in the storage unit 30, by using the temporary storage unit 50 (B2). For example, the banknote handling apparatus 1 performs an inventory confirming process of recognizing and disinfecting the banknotes being stored in the storage unit 30. The inventory confirming process is performed to confirm that the denomination and the number of banknotes being stored in the storage unit 30 match banknote information, of the storage unit 30, managed in the memory 70. The inventory confirming process is sometimes called a reconciliation process.

In the inventory confirming process in the mode of performing the banknote recognition and the banknote disinfection, the banknotes fed out from the storage unit 30 one by one to the transport unit 40 are directly transported to the disinfection unit 10 without passing the recognition unit 20. After the banknotes have been disinfected by the disinfection unit 10, these banknotes are recognized by the recognition unit 20, transported to the temporary storage unit 50 and stored therein temporarily. After all the banknotes in the storage unit 30 have been temporarily stored in the temporary storage unit 50, the temporary storage unit 50 feeds out all the temporarily stored banknotes to the transport unit 40 one by one. Each fed-out banknote is transported to the recognition unit 20 and recognized by the recognition unit 20. After being disinfected by the disinfection unit 10, each banknote is transported to the storage unit 30 and stored therein. The banknote recognition of each banknote by the recognition unit 20 may be performed on the way to the temporary storage unit 50 from the storage unit 30 via the disinfection unit 10, and/or on the way to the storage unit 30 from the temporary storage unit 50 via the disinfection unit 10. The banknote disinfection of each banknote may be performed only once on the way to the temporary storage unit 50 from the storage unit 30, or on the way to the storage unit 30 from the temporary storage unit 50. The banknote handling apparatus 1 can confirm that the banknotes stored in the storage unit 30 match the banknote information, of the storage unit 30, managed in the memory 70, based on the recognition result by the recognition unit 20. The banknote handling apparatus 1 may control the entire inventory confirming process as one banknote handling process. Alternatively, the banknote handling apparatus 1 may control the process performed while banknotes are transported to the temporary storage unit 50 from the storage unit 30 and the other process performed while the banknotes are returned to the storage unit 30 from the temporary storage unit 50, as separated banknote handling processes.

The banknote handling apparatus 1 can perform a banknote handling process of moving the banknotes in the storage unit 30 to inside the apparatus 1, in a mode of disinfecting the banknotes without recognizing them. Such a banknote handling process is referred to as an intra-apparatus disinfection process. While the aforementioned disinfection process is a process of disinfecting the banknotes received in the depositing unit 11 and discharging the disinfected banknotes from the banknote handling apparatus 1 through the dispensing unit 12, the intra-apparatus disinfection process is a process of disinfecting, inside the apparatus 1, the banknotes that have been stored in the storage unit 30 without discharging them from the apparatus 1.

In the intra-apparatus disinfection process, the banknotes, having been fed out from the storage unit 30 one by one to the transport unit 40, are directly transported to the disinfection unit 10 without passing the recognition unit 20, and disinfected by the disinfection unit 10. Thereafter, these banknotes are transported to the temporary storage unit 50 via the recognition unit 20 and stored therein temporarily. After all the banknotes to be disinfected have been temporarily stored in the temporary storage unit 50, the temporary storage unit 50 feeds out all the temporarily stored banknotes to the transport unit 40 one by one. The fed-out banknotes are transported to the disinfection unit 10 via the recognition unit 20, disinfected by the disinfection unit 10, and stored in the storage unit 30. Even when the temporary storage unit 50 is used for the intra-apparatus disinfection process, the banknotes may be disinfected only once on the way to the temporary storage unit 50 from the storage unit 30, or on the way to the storage unit 30 from the temporary storage unit 50.

The banknote handling apparatus 1 can change the banknote transport speed (C1). A value of the banknote transport speed is not particularly limited. For example, the banknote handling apparatus 1 transports banknotes at 800 mm/sec for low-speed transport, and transports banknotes at 1600 mm/sec for high speed transport.

The banknote handling apparatus 1 can change the banknote transport speed for each banknote handling process. For example, the banknote handling apparatus 1 sets the banknote transport speed to be lower in the banknote handling process in the mode of disinfecting banknotes than in the banknote handling process in the mode of not disinfecting banknotes. In the banknote handling process in the mode of disinfecting banknotes, the disinfection unit 10 irradiates the banknotes being transported at the low speed with the ultraviolet ray, thereby enhancing the disinfection effect.

The banknote handling apparatus 1 can change the banknote transport speed for each of the plurality of types of banknote handling process performed in the mode of disinfecting banknotes. For example, the banknote handling apparatus 1 sets the transport speed to be higher in the dispensing process in the disinfection mode than in the depositing process in the disinfection mode. In a so-called recycle type banknote handling apparatus 1, the dispensing process is performed by dispensing banknotes that have been stored in the storage unit 30 in the depositing process. There is a high possibility that each banknote to be dispensed in the dispensing process has been disinfected at least once when it was deposited in the depositing process. Therefore, the time required for the dispensing process can be reduced by transporting the banknote, which is highly likely to have been disinfected, at a higher speed in the dispensing process than in the depositing process.

When the same banknotes are disinfected a plural number of times during one banknote handling process, the banknote handling apparatus 1 can change the banknote transport speed for each disinfection. For example, in the depositing process in the disinfection mode using the temporary storage unit 50, first banknote disinfection is performed while the banknotes received in the depositing unit 11 are transported to the temporary storage unit 50 in order to store these banknotes therein temporarily, and second banknote disinfection is performed while the banknotes temporarily stored in the temporary storage unit 50 are transported to the storage unit 30 in order to store these banknotes therein. The banknote handling apparatus 1 can set the banknote transport speed for one of the two times of disinfection to be higher than that for the other disinfection. For example, the banknote handling apparatus 1 transports the banknotes at a high speed during the first banknote disinfection, and transports, during the second banknote disinfection, the banknotes at a speed lower than that in the first banknote disinfection.

For example, in the depositing process in the mode of performing the banknote recognition and the banknote disinfection, an operation of approving the recognition result is performed by the operator in a state where the banknotes that have been subjected to the banknote recognition and the first banknote disinfection are stored in the temporary storage unit 50 temporarily. After the operator has approved the recognition result, the depositing process is finished, and then the banknotes in the temporary storage unit 50 are subjected to the second banknote disinfection and stored in the storage unit 30. Therefore, the time required for the depositing process can be reduced by setting the banknote transport speed for the first banknote disinfection to be higher than that for the second banknote disinfection, and the banknote disinfection effect can be enhanced by setting the banknote transport speed for the second banknote disinfection to be lower.

The banknote handling apparatus 1 can perform the banknote handling process in the mode of not disinfecting banknotes. Specifically, the banknote handling apparatus 1 can perform the banknote handling processes such as the aforementioned depositing process, dispensing process, counting process, and inventory confirming process, without performing the banknote disinfection by the disinfection unit 10. That is, these banknote handling processes can be performed with the light sources of the disinfection unit 10 being turned off. In the banknote handling process performed with the light sources being turned off, the banknotes may be transported at a higher speed than that of another banknote handling process performed with the light sources being turned on.

The banknote handling apparatus 1 can perform the banknote handling process in a mode selected from among a plurality of types of modes. The mode of the banknote handling process may be selected by the operator through the operation unit 81, or may be selected by the external device through the communication unit 90.

The modes selectable in the banknote handling apparatus 1 include: a first mode of performing both the banknote recognition and the banknote disinfection; a second mode of performing the banknote disinfection without performing the banknote recognition; and a third mode of performing the banknote recognition without performing the banknote disinfection.

The banknote handling process in the first mode of performing both the banknote recognition and the banknote disinfection, includes at least one of the depositing process, the dispensing process, the counting process, and the inventory confirming process which are performed with the light sources of the disinfection unit 10 being turned on. The banknote handling process in the second mode of performing the banknote disinfection without performing the banknote recognition, includes at least one of the dispensing process in which dispensed banknotes are not recognized, the disinfection process, and the intra-apparatus disinfection process. The banknote handling process in the third mode of performing the banknote recognition without performing the banknote disinfection, includes at least one of the depositing process, the dispensing process, the counting process, and the inventory confirming process which are performed with the light sources of the disinfection unit 10 being turned off. The mode of the banknote handling process to be performed by the banknote handling apparatus 1 is selected from among the above modes, whereby the banknotes can be handled and disinfected efficiently.

The banknote handling apparatus 1 may not necessarily perform all the processes described with reference to FIG. 1, and may perform only a part of these processes according to the usage method and the configuration of the banknote handling apparatus 1. Hereinafter, a specific example of the banknote handling apparatus 1 will be described. The description will be mainly given for the configuration and operation of the banknote handling unit 2 while the control unit 60, the memory 70, the operation unit 81, the display 82, and the communication unit 90 are omitted in the drawings showing the configuration of the apparatus.

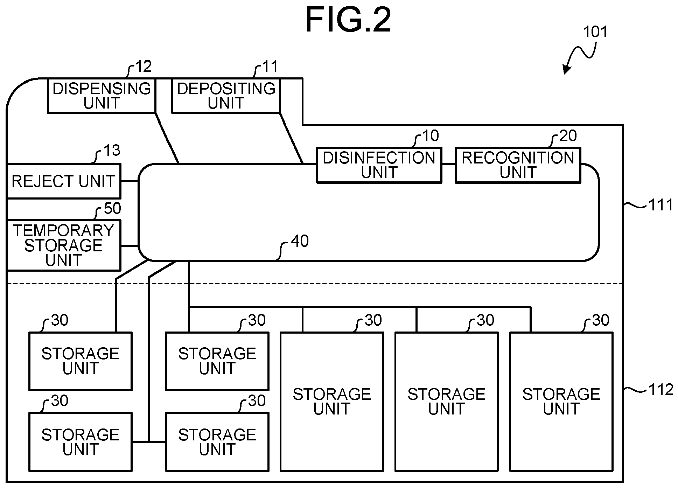

FIG. 2 shows an example of a banknote handling apparatus 101. The banknote handling apparatus 101 includes a reject unit 13 in addition to the components shown in FIG. 1. For example, among banknotes received in the depositing unit 11 for the depositing process, a reject banknote, which is determined not to be deposited based on the recognition result by the recognition unit 20, is stored in the reject unit 13. The reject banknote can be taken out of the banknote handling apparatus 101 from the reject unit 13.

The banknote handling apparatus 101 is divided into an upper unit 111 and a lower unit 112. The lower unit 112 is a safe. A plurality of storage units 30 for storing a large number of banknotes are disposed in the safe. The transport unit 40 can transport banknotes between the upper unit 111 and the lower unit 112.

The banknote handling apparatus 101 can perform a depositing process not using the temporary storage unit 50, and/or a depositing process using the temporary storage unit 50. The operator of the banknote handling apparatus 101 can change the type of the depositing process to be performed by the banknote handling apparatus 101. The operator who has started the depositing process on the banknote handling apparatus 101 puts, in the depositing unit 11, one or a plurality of banknotes to be deposited.

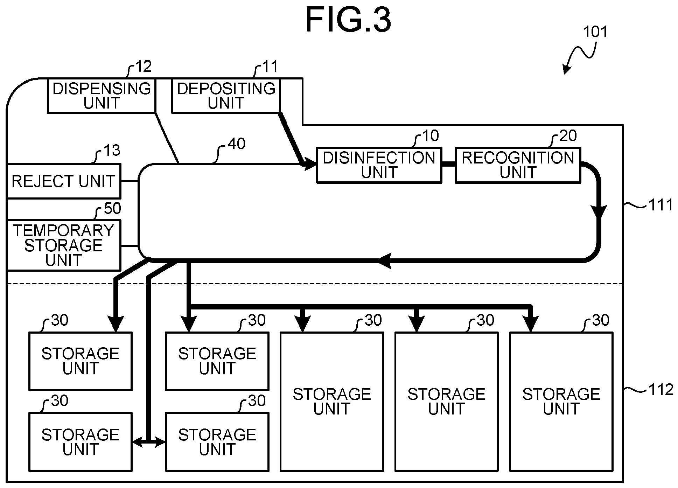

FIG. 3 illustrates the depositing process in the first mode not using the temporary storage unit 50. The depositing unit 11 feeds out the banknotes one by one to the transport unit 40 inside the banknote handling apparatus 101. The transport unit 40 transports each banknote as indicated by arrows in FIG. 3.

The transport unit 40 transports each banknote fed out from the depositing unit 11, to the disinfection unit 10. The disinfection unit 10 disinfects each banknote being transported by the transport unit 40. The transport unit 40 transports each banknote from the disinfection unit 10 to the recognition unit 20. The recognition unit 20 recognizes each banknote being transported by the transport unit 40. The transport unit 40 transports each banknote from the recognition unit 20 into the safe in the lower unit 112. Each banknote is stored in any of the plurality of storage units 30 based on the recognition result. For example, each storage unit 30 is assigned to a denomination of banknotes to be stored therein, so that the banknotes are stored in the plurality of storage units 30 by denomination.

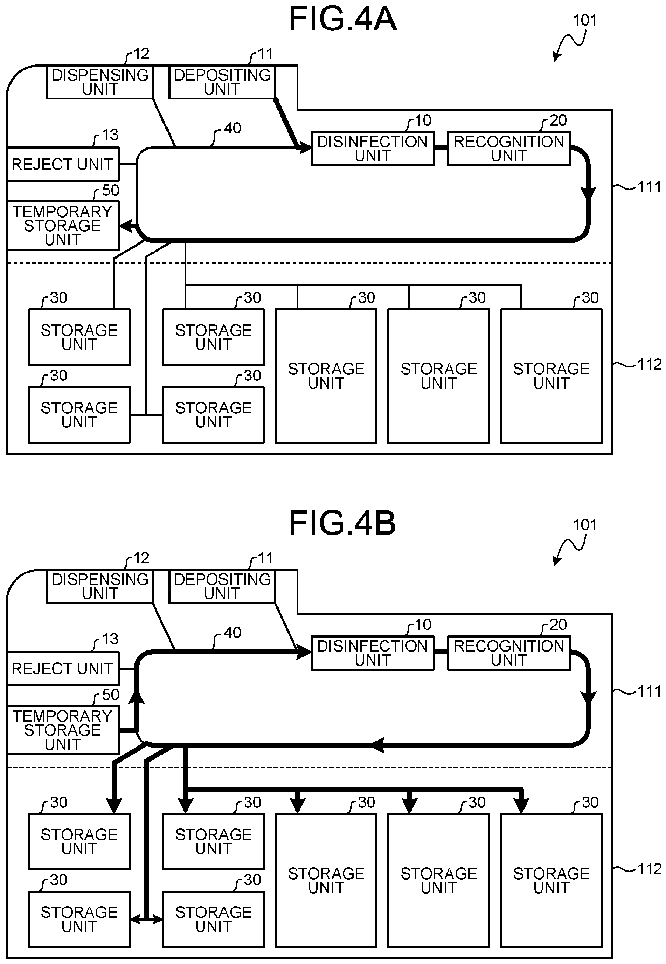

FIGS. 4A and 4B illustrate the depositing process in the first mode using the temporary storage unit 50. The depositing process in the first mode using the temporary storage unit 50 includes: a temporary storage process of disinfecting the banknotes fed from the depositing unit 11, and temporarily storing these banknotes in the temporary storage unit 50; and a storage process of disinfecting the banknotes fed out from the temporary storage unit 50, and storing these banknotes in the storage unit 30. In the depositing process using the temporary storage unit 50, each banknote is disinfected twice during one depositing process.

FIG. 4A shows movement of the banknotes in the temporary storage process. The depositing unit 11 feeds out the banknotes one by one to the transport unit 40 inside the banknote handling apparatus 101. The transport unit 40 transports each banknote to the disinfection unit 10. The disinfection unit 10 disinfects each banknote being transported by the transport unit 40. The transport unit 40 transports each banknote from the disinfection unit 10 to the recognition unit 20. The recognition unit 20 recognizes each banknote being transported by the transport unit 40. The transport unit 40 transports each banknote from the recognition unit 20 to the temporary storage unit 50 in the upper unit 111. The temporary storage unit 50 temporarily stores each banknote therein. After all the banknotes in the depositing unit 11 have been temporarily stored in the temporary storage unit 50, the storage process of storing these banknotes into the storage unit 30 is started.

After the temporary storage process has been completed, the recognition result of the banknotes temporarily stored in the temporary storage unit 50 is displayed on the display 82. The recognition result includes the total amount of the banknotes temporarily stored in the temporary storage unit 50, that is, the amount of the banknotes to be deposited. Upon confirming the information on the display 82, the operator operates the operation unit 81 to approve the recognition result and finish the depositing process. Then, the storage process is started.

FIG. 4B shows movement of the banknotes in the storage process. The temporary storage unit 50 feeds out all the temporarily stored banknotes to the transport unit 40 one by one. The transport unit 40 transports each banknote from the temporary storage unit 50 to the disinfection unit 10. The disinfection unit 10 disinfects each banknote being transported by the transport unit 40. The transport unit 40 transports each banknote from the disinfection unit 10 to the recognition unit 20. The recognition unit 20 recognizes each banknote being transported by the transport unit 40. The transport unit 40 transports each banknote from the recognition unit 20 into the safe in the lower unit 112. Each banknote is stored in any of the plurality of storage units 30 based on the recognition result. Thus, all the banknotes temporarily stored in the temporary storage unit 50, that is, all the deposited banknotes, are stored in the storage unit 30.

In the above example, the recognition unit 20 recognizes the banknotes in both the temporary storage process of transporting the banknotes from the depositing unit 11 to the temporary storage unit 50 in order to store the banknotes therein temporarily, and the storage process of transporting the banknotes from the temporary storage unit 50 to the storage unit 30 in order to store the banknotes therein. However, the recognition unit 20 may recognize the banknotes only once in the temporary storage process or the storage process. The banknote handling apparatus 101 may control the temporary storage process and the storage process as one banknote handling process or as different banknote handling processes.

The banknote handling apparatus 101 can perform a dispensing process not using the temporary storage unit 50, and/or a dispensing process using the temporary storage unit 50. The operator of the banknote handling apparatus 101 can change the type of the dispensing process to be performed by the banknote handling apparatus 101. For example, the operator operates the operation unit 81 to start the dispensing process and designate banknotes to be dispensed. Alternatively, for example, information designating banknotes to be dispensed is inputted from the external device to the banknote handling apparatus 101 through the communication unit 90.

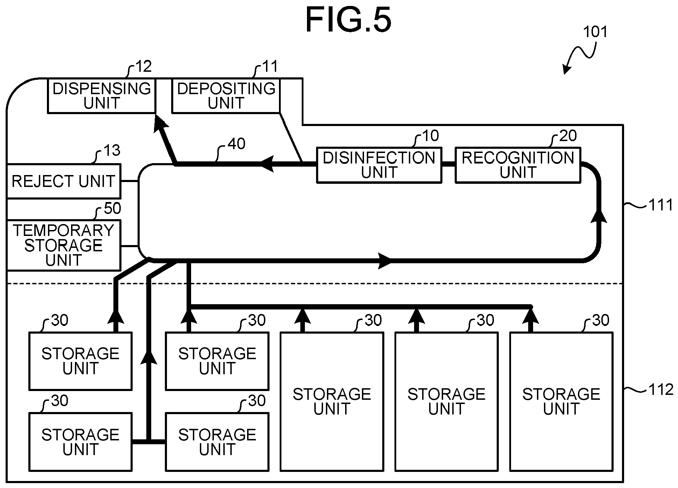

FIG. 5 illustrates the dispensing process in the first mode not using the temporary storage unit 50. Banknotes to be dispensed are fed out from one or a plurality of storage units 30 one by one to the transport unit 40. The transport unit 40 transports the banknotes as shown by arrows in FIG. 5.

The transport unit 40 transports each banknote fed out from the storage unit 30, one by one, to the recognition unit 20 in the upper unit 111. The recognition unit 20 recognizes each banknote being transported by the transport unit 40. The transport unit 40 transports each banknote from the recognition unit 20 to the disinfection unit 10. The disinfection unit 10 disinfects each banknote being transported by the transport unit 40. The transport unit 40 transports each banknote from the disinfection unit 10 to the dispensing unit 12. The dispensing unit 12 stacks each banknote received from the transport unit 40. Thus, all the banknotes to be dispensed are discharged to the dispensing unit 12.

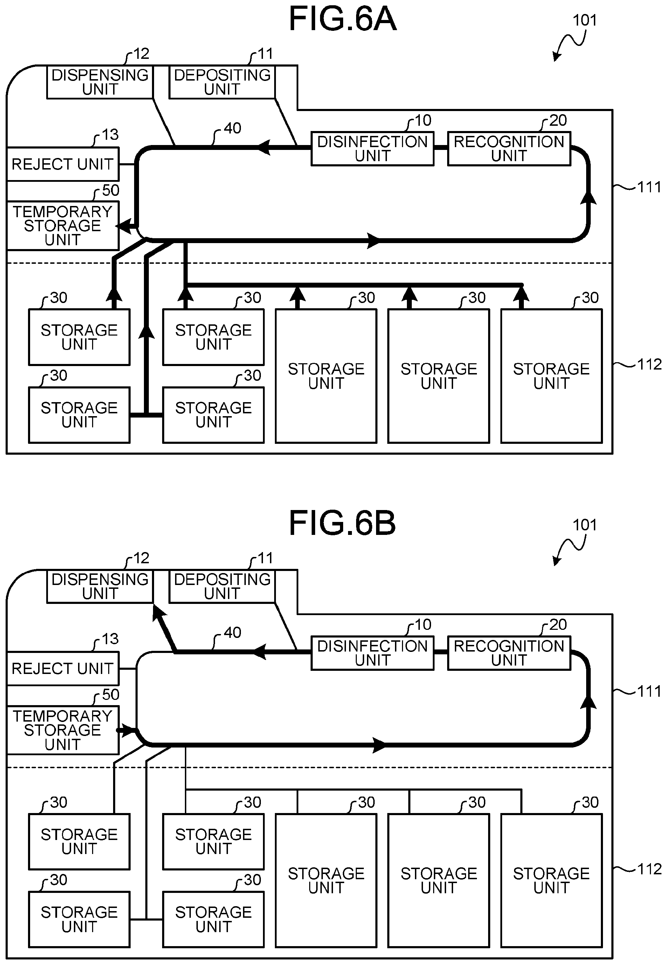

FIGS. 6A and 6B illustrate the dispensing process in the first mode using the temporary storage unit 50. The dispensing process in the first mode using the temporary storage unit 50 includes: a temporary storage process of disinfecting the banknotes fed out from the storage unit 30, and temporarily storing these banknotes in the temporary storage unit 50; and a discharging process of disinfecting the banknotes fed out from the temporary storage unit 50, and discharging these banknotes to the dispensing unit 12. In the dispensing process using the temporary storage unit 50, each banknote is disinfected twice during one dispensing process.

FIG. 6A shows movement of the banknotes in the temporary storage process. Banknotes to be dispensed are fed out from one or a plurality of storage units 30 to the transport unit 40 one by one. The transport unit 40 transports each banknote fed out from the storage unit 30, one by one to the recognition unit 20 in the upper unit 111. The recognition unit 20 recognizes each banknote being transported by the transport unit 40. The transport unit 40 transports each banknote from the recognition unit 20 to the disinfection unit 10. The disinfection unit 10 disinfects each banknote being transported by the transport unit 40. The transport unit 40 transports each banknote from the disinfection unit 10 to the temporary storage unit 50. The temporary storage unit 50 stores each banknote therein temporarily. After all the banknotes to be dispensed have been temporarily stored in the temporary storage unit 50, the discharging process of discharging these banknotes to the dispensing unit 12 is started.

FIG. 6B shows movement of the banknotes in the discharging process. The temporary storage unit 50 feeds out all the temporarily stored banknotes to the transport unit 40 one by one. The transport unit 40 transports each banknote from the temporary storage unit 50 to the recognition unit 20. The recognition unit 20 recognizes each banknote being transported by the transport unit 40. The transport unit 40 transports each banknote from the recognition unit 20 to the disinfection unit 10. The disinfection unit 10 disinfects each banknote being transported by the transport unit 40. The transport unit 40 transports each banknote from the disinfection unit 10 to the dispensing unit 12. The dispensing unit 12 stacks each banknote received from the transport unit 40. Thus, all the banknotes temporarily stored in the temporary storage unit 50, that is, all the banknotes to be dispensed, are dispensed from the dispensing unit 12.

In the above example, the recognition unit 20 recognizes the banknotes in both the temporary storage process of transporting the banknotes from the storage unit 30 to the temporary storage unit 50 in order to store the banknotes therein, and the discharging process of transporting the banknotes from the temporary storage unit 50 to the dispensing unit 12 in order to dispense the banknotes therefrom. However, the recognition unit 20 may recognize the banknotes only once in the temporary storage process or the discharging process. The banknote handling apparatus 101 may control the temporary storage process and the discharging process as one banknote handling process or as different banknote handling processes.

The banknote handling apparatus 101 can perform the inventory confirming process by using the temporary storage unit 50. The banknote handling apparatus 101 manages, in the memory 70, information on the banknotes being stored in each of the plurality of storage units 30, as inventory information. For example, the inventory information includes the denomination of the banknotes stored in each of the storage units 30, and the number of banknotes for each denomination. The inventory confirming process is a process for confirming that the information on the banknotes actually stored in the storage unit 30 matches the inventory information. All the banknotes are fed out from one storage unit 30 one by one and recognized by the recognition unit 20, thereby confirming that the information on the banknotes being stored in the storage unit 30 matches the inventory information.

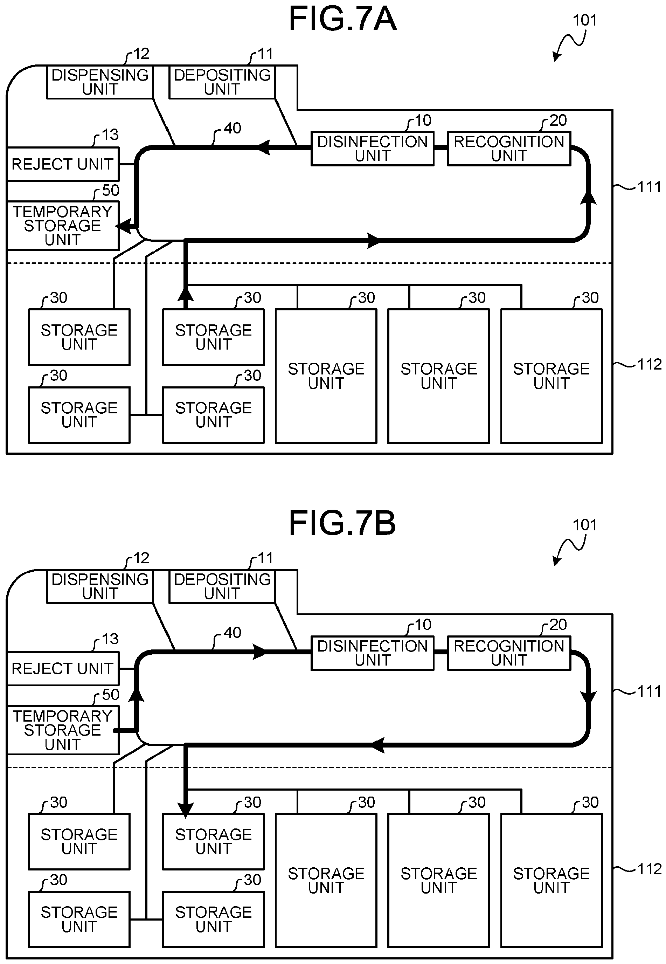

FIGS. 7A and 7B illustrate the inventory confirming process in the first mode. Although FIGS. 7A and 7B show movement of banknotes in the inventory confirming process of one storage unit 30, the inventory confirming processes of the other storage units 30 are similarly performed. The inventory confirming process includes: a temporary storage process of feeding out the banknotes from the storage unit 30, and storing these banknotes in the temporary storage unit 50 temporarily; and a returning process of feeding out the banknotes from the temporary storage unit 50, and returning these banknotes to the storage unit 30. Each banknote is disinfected twice during one inventory confirming process.

FIG. 7A shows movement of the banknotes in the temporary storage process. The storage unit 30 feeds out all the banknotes stored therein, one by one, to the transport unit 40. The transport unit 40 transports each banknote fed out from the storage unit 30 to the recognition unit 20. The recognition unit 20 recognizes each banknote being transported by the transport unit 40. The transport unit 40 transports each banknote from the recognition unit 20 to the disinfection unit 10. The disinfection unit 10 disinfects each banknote being transported by the transport unit 40. The transport unit 40 transports each banknote from the disinfection unit 10 to the temporary storage unit 50. The temporary storage unit 50 stores each banknote temporarily. After all the banknotes in the storage unit 30 have been temporarily stored in the temporary storage unit 50, the returning process of returning these banknotes to the storage unit 30 is started.

FIG. 7B shows movement of the banknotes in the returning process. The temporary storage unit 50 feeds out all the temporarily stored banknotes to the transport unit 40 one by one. The transport unit 40 transports each banknote from the temporary storage unit 50 to the disinfection unit 10. The disinfection unit 10 disinfects each banknote being transported by the transport unit 40. The transport unit 40 transports each banknote from the disinfection unit 10 to the recognition unit 20. The recognition unit 20 recognizes each banknote being transported by the transport unit 40. The transport unit 40 transports each banknote from the recognition unit 20 to the original storage unit 30 in which the banknotes were stored. The storage unit 30 stores each banknote received from the transport unit 40. Thus, all the banknotes temporarily stored in the temporary storage unit 50 are returned to the original storage unit 30. The banknote handling apparatus 101 confirms that the recognition result of the banknotes in the storage unit 30 matches the inventory information in the memory 70. For example, the banknote handling apparatus 101 displays the confirmation result on the display 82.

In the above example, the recognition unit 20 recognizes the banknotes in both the temporary storage process of transporting the banknotes from the storage unit 30 to the temporary storage unit 50 in order to store the banknotes therein, and the returning process of returning the banknotes from the temporary storage unit 50 to the storage unit 30. However, the recognition unit 20 may recognize the banknotes only once in the temporary storage process or the returning process. The inventory confirming process may not necessarily be performed by using the temporary storage unit 50, and may be performed by using another structure capable of performing storage and feeding-out of banknotes. For example, one of the storage units 30 may be used for the inventory confirming process so that the banknotes are temporarily stored in this storage unit 30 during the temporary storage process. The banknote handling apparatus 101 may control the temporary storage process and the returning process as one banknote handling process or as different banknote handling processes.

In the inventory confirming process, the banknote handling apparatus 101 disinfects each banknote stored in the storage unit 30 twice, however, the banknote may be further disinfected by repeating the temporary storage process and the returning process shown in FIGS. 7A and 7B. The banknote handling apparatus 101 can repeat disinfection for the same banknote a predetermined number of times, or a number of times that the operator has specified on the operation unit 81 at the time of starting the inventory confirming process. For example, a banknote is disinfected four times if the temporary storage process and the returning process are repeated twice in one inventory confirming process.

The banknote handling apparatus 101 can perform the intra-apparatus disinfection process. The intra-apparatus disinfection process is a process of disinfecting banknotes inside the apparatus. The intra-apparatus disinfection process is performed by: disinfecting each banknote, which is fed out from the storage unit 30, by the disinfection unit 10 and temporarily storing each banknote in the temporary storage unit 50 as shown in FIG. 7A; and disinfecting each banknote, which is fed out from the temporary storage unit 50, again by the disinfection unit 10 and returning each banknote to the storage unit 30 as shown in FIG. 7B. Although movement of the banknotes is the same as that in the inventory confirming process, the intra-apparatus disinfection process is performed in the second mode in which the banknote recognition by the recognition unit 20 is not performed.

The intra-apparatus disinfection process in the second mode can be manually started by the operator performing a predetermined operation on the operation unit 81. When the operator instructs to execute the intra-apparatus disinfection process, the banknote handling apparatus 101 disinfects all the banknotes stored in all the storage units 30. Alternatively, the operator can start the intra-apparatus disinfection process while designating one or a plurality of storage units 30 so that only the banknotes in the designated storage unit 30 are disinfected.

In contrast to the inventory confirming process, in the intra-apparatus disinfection process, only a part of the banknotes being stored in the storage unit 30 can be fed out and disinfected. For example, it is possible to disinfect only a predetermined number (e.g., 10) or a predetermined percentage (e.g., 10%) of banknotes from among all the banknotes stored in the storage unit 30. The number or percentage of banknotes to be disinfected can be set in advance, or the operator can designate the same when starting the intra-apparatus disinfection process.

In the banknote handling apparatus 101, the banknote transport speed can be changed between the intra-apparatus disinfection process and the other banknote handling processes. For example, banknotes are transported at a high speed to reduce the handling time in the depositing process, the dispensing process, and the inventory confirming process, while banknotes are transported at a low speed to enhance the disinfection effect in the intra-apparatus disinfection process. In the case where the banknote handling apparatus 101 is used for transactions with customers in a retail store or a branch of a financial institution, when a banknote received from a customer is deposited to the apparatus 101, this banknote is transported at a high speed, whereas the intra-apparatus disinfection process is performed at a low transport speed when there is no customer. Thus, banknotes can be disinfected more reliably in the intra-apparatus disinfection process while the customer being served is not kept waiting the depositing process. Likewise, in the process of dispensing a banknote to be handed to the customer, the banknote is transported at a higher speed than that in the intra-apparatus disinfection process, whereby the process can be completed within a shorter time.

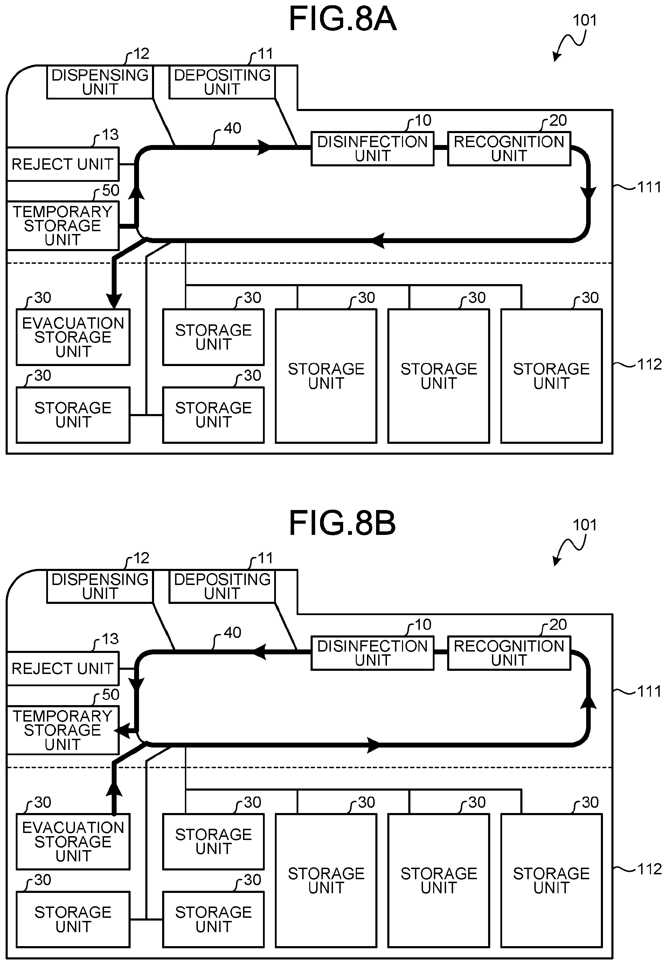

The banknote handling apparatus 101 can repeatedly disinfect banknotes by using one of the plurality of storage units 30 as an evacuation storage unit in which banknotes are evacuated and stored. FIGS. 8A and 8B illustrate a method for repeating disinfection of banknotes.

When the depositing process in the first mode is performed by using the temporary storage unit 50 as shown in FIGS. 4A and 4B, the banknote handling apparatus 101 temporarily stores the banknotes in the temporary storage unit 50 as shown in FIG. 4A, and thereafter completes the depositing process upon receiving an approval from the operator. Thereafter, the banknote handling apparatus 101 feeds out the banknotes from the temporary storage unit 50, disinfects the banknotes by the disinfection unit 10, and stores these banknotes in the evacuation storage unit 30 as shown in FIG. 8A. After all the banknotes in the temporary storage unit 50 have been disinfected and stored in the evacuation storage unit 30, the banknote handling apparatus 101 feeds out the banknotes from the evacuation storage unit 30, disinfects the banknotes by the disinfection unit 10, and returns the banknotes to the temporary storage unit 50 again as shown in FIG. 8B. After all the banknotes in the evacuation storage unit 30 have been returned to the temporary storage unit 50, the banknote handling apparatus 101 stores the banknotes in the storage unit 30 as shown in FIG. 4B to complete the depositing process. As a result, each banknote is disinfected four times during one depositing process. The banknote handling apparatus 1 can further disinfect the same banknote by repeating the handling process shown in FIGS. 8A and 8B.

Also, in the dispensing process in the first mode illustrated in FIGS. 6A and 6B and in the inventory confirming process in the first mode and the intra-apparatus disinfection process in the second mode illustrated in FIGS. 7A and 7B, after the banknotes are temporarily stored in the temporary storage unit 50, the banknotes are moved between the temporary storage unit 50 and the evacuation storage unit 30 via the disinfection unit 10 as shown in FIGS. 8A and 8B, whereby the banknotes can be repeatedly disinfected. The operator can change the number of times of disinfection to be performed in each banknote handling process by using the operation unit 81.

The banknote handling apparatus 101 can perform the counting process in the first mode. The counting process is a banknote handling process of feeding out the banknotes received in the depositing unit 11 into the apparatus, subjecting the banknotes to disinfection by the disinfection unit 10 and recognition by the recognition unit 20, and returning the banknotes from the dispensing unit 12 without storing the banknotes in the storage unit 30. The banknote handling apparatus 101 can perform the counting process not using the temporary storage unit 50, and/or the counting process using the temporary storage unit 50. The operator of the banknote handling apparatus 101 can change the type of the counting process to be performed by the banknote handling apparatus 101. The operator having started the counting process puts a plurality of banknotes to be counted, in the depositing unit 11.

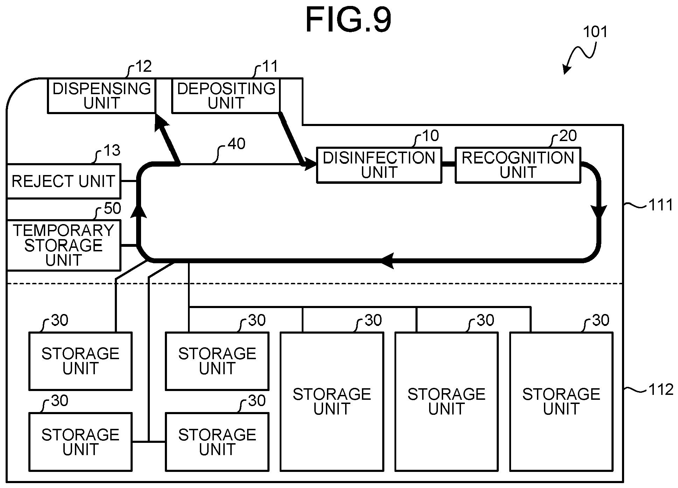

FIG. 9 illustrates the counting process in the first mode not using the temporary storage unit 50. The depositing unit 11 feeds out the banknotes to the transport unit 40 inside the apparatus one by one. The transport unit 40 transports each banknote fed out from the depositing unit 11 to the disinfection unit 10. The disinfection unit 10 disinfects each banknote being transported by the transport unit 40. The transport unit 40 transports each banknote from the disinfection unit 10 to the recognition unit 20. The recognition unit 20 recognizes each banknote being transported by the transport unit 40. The transport unit 40 transports each banknote from the recognition unit 20 to the dispensing unit 12. The dispensing unit 12 stacks each banknote received from the transport unit 40. After all the banknotes in the depositing unit 11 have been recognized, the banknote handling apparatus 101 displays the recognition result of the banknotes on the display 82. For example, the number of banknotes for each denomination, the total number of banknotes, and the total amount of banknotes are displayed on the display 82. The operator confirms the recognition result of the banknotes, and takes out the banknotes disinfected in the apparatus from the dispensing unit 12.

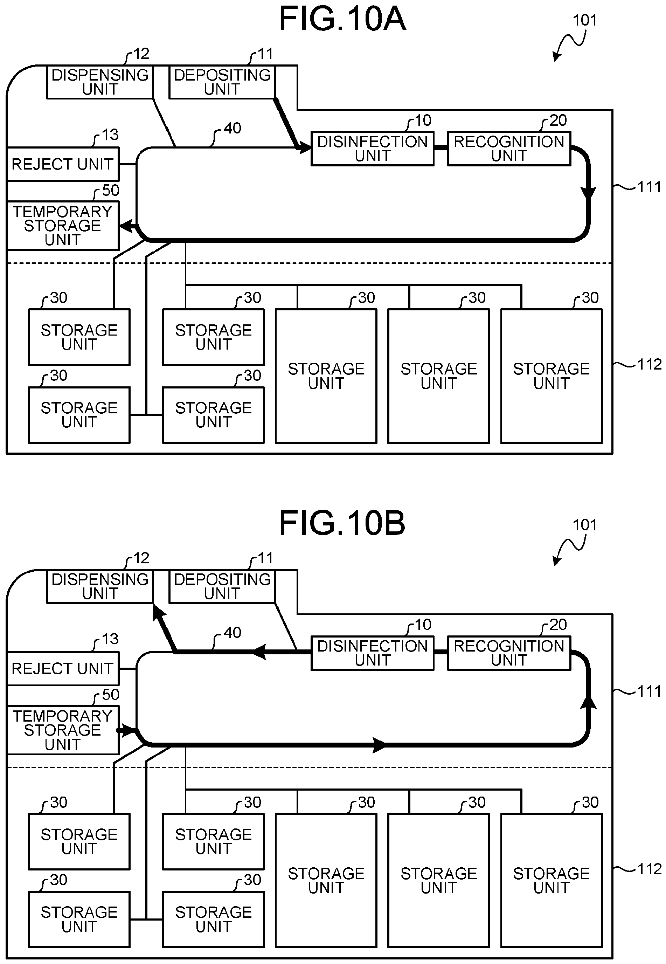

FIGS. 10A and 10B illustrate the counting process in the first mode using the temporary storage unit 50. The counting process using the temporary storage unit 50 includes: a temporary storage process of feeding out banknotes from the depositing unit 11 and temporarily storing the banknotes in the temporary storage unit 50; and a discharging process of feeding out the banknotes from the temporary storage unit 50 and discharging the banknotes to the dispensing unit 12.

FIG. 10A shows movement of the banknotes in the temporary storage process. The depositing unit 11 feeds out the banknotes to the transport unit 40 inside the apparatus one by one. The transport unit 40 transports each banknote to the disinfection unit 10. The disinfection unit 10 disinfects each banknote being transported by the transport unit 40. The transport unit 40 transports each banknote from the disinfection unit 10 to the recognition unit 20. The recognition unit 20 recognizes each banknote being transported by the transport unit 40. The transport unit 40 transports each banknote from the recognition unit 20 to the temporary storage unit 50 in the upper unit 111. The temporary storage unit 50 stores each banknote therein temporarily. After all the banknotes in the depositing unit 11 have been temporarily stored in the temporary storage unit 50, the discharging process of discharging the banknotes from the temporary storage unit 50 to the dispensing unit 12 is started.

FIG. 10B shows movement of the banknotes in the discharging process. The temporary storage unit 50 feeds out all the temporarily stored banknotes to the transport unit 40 one by one. The transport unit 40 transports each banknote from the temporary storage unit 50 to the recognition unit 20. The recognition unit 20 recognizes each banknote being transported by the transport unit 40. The transport unit 40 transports each banknote from the recognition unit 20 to the disinfection unit 10. The disinfection unit 10 disinfects each banknote being transported by the transport unit 40. The transport unit 40 transports each banknote from the disinfection unit 10 to the dispensing unit 12. The dispensing unit 12 stacks each banknote received from the transport unit 40. Thus, all the banknotes temporarily stored in the temporary storage unit 50 are discharged to the dispensing unit 12. In the same way as in the case where the temporary storage unit 50 is not used for the counting process, the banknote handling apparatus 101 displays, on the display 82, the recognition result including the total amount of the banknotes.

In the above example, the recognition unit 20 recognizes the banknotes in both the temporary storage process of transporting the banknotes from the depositing unit 11 to the temporary storage unit 50 in order to store the banknotes therein, and the discharging process of discharging the banknotes from the temporary storage unit 50 to the dispensing unit 12. However, the recognition unit 20 may recognize the banknotes only once in the temporary storage process or the discharging process. The banknote handling apparatus 101 may control the temporary storage process and the discharging process as one banknote handling process or as different banknote handling processes.

The banknote handling apparatus 101 can perform the disinfection process in the second mode. The disinfection process in the second mode is a banknote handling process of feeding out the banknotes received in the depositing unit 11 into inside the apparatus, disinfecting the banknotes by the disinfection unit 10 without performing recognition by the recognition unit 20, and returning the banknotes from the dispensing unit 12 without storing the banknotes in the storage unit 30. Since the banknotes are handled in the same manner as the counting process in the first mode except that the banknote recognition by the recognition unit 20 is not performed, the detailed description thereof is omitted.

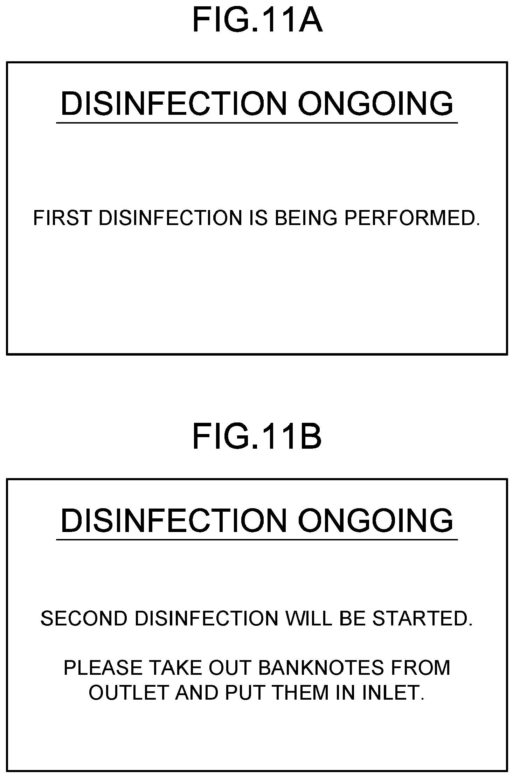

The banknote handling apparatus 101 can display, on the display 82, information indicating that the banknotes are disinfected inside the apparatus. FIGS. 11A and 11B each show an example of a screen displayed on the display 82. For example, in the counting process shown in FIG. 9 and in the temporary storage process in the counting process shown in FIG. 10A, information indicating that the first disinfection of the banknotes is being performed is displayed on the display 82 as shown in FIG. 11A. In the case where the banknotes are to be disinfected twice in the counting process using the temporary storage unit 50, information indicating that the second disinfection of the banknotes is being performed is displayed on the display 82 when starting the discharging process shown in FIG. 10B.

Similarly, when the other banknote handling processes such as the depositing process, the dispensing process, the inventory confirming process, the intra-apparatus disinfection process, the disinfection process, and the counting process is performed, the banknote handling apparatus 101 can display, on the display 82, information indicating that banknotes are being disinfected and/or information indicating the number of times of disinfection, in order to notify the operator of the information

When performing the counting process in the first mode without using the temporary storage unit 50 shown in FIG. 9., the banknote handling apparatus 101 can also disinfect the banknotes a plural number of times. The number of times of banknote disinfection can be set in advance, or the operator can designate the same when instructing execution of the counting process.

For example, when disinfecting the banknotes twice in the counting process in the first mode not using the temporary storage unit 50, the banknote handling apparatus 101 performs the process shown in FIG. 9 to subject the banknotes to the first banknote disinfection. In the first banknote disinfection, the banknote handling apparatus 101 displays the screen shown in FIG. 11A on the display 82. After all the banknotes in the depositing unit 11 have been subjected to recognition and disinfection and discharged to the dispensing unit 12, the banknote handling apparatus 101 displays the screen shown in FIG. 11B on the display 82. Information instructing the operator to take out the banknotes from the dispensing unit 12 and put the banknotes in the depositing unit 11, is displayed on the screen. Upon confirming the instruction on the display 82, the operator moves the banknotes from the dispensing unit 12 to the depositing unit 11. Thereafter, the process shown in FIG. 9 is started again, and the banknotes are subjected to the second banknote disinfection. For example, after moving the banknotes from the dispensing unit 12 to the depositing unit 11, the operator performs a predetermined operation on the operation unit 81 to start the second banknote disinfection. Alternatively, for example, a sensor disposed in the depositing unit 11 detects the banknotes placed in the depositing unit 11 by the operator, thereby automatically starting the second banknote disinfection. The banknote handling apparatus 101 displays a screen similar to that shown in FIG. 11A on the display 82 to notify the operator that the second banknote disinfection is being performed. After all the banknotes in the depositing unit 11 have been recognized, the banknote handling apparatus 101 displays, on the display 82, the recognition result including the total amount of the banknotes, thereby completing the counting process. Similarly, in the disinfection process in the second mode, the operator moves the banknotes in the dispensing unit 12 to the depositing unit 11, whereby the banknotes can be disinfected a plural number of times.

When performing the counting process in the first mode using the temporary storage unit 50 as shown in FIGS. 10A and 10B, the banknote handling apparatus 101 can disinfect the banknotes three or more times. For example, when disinfecting the banknotes are disinfected three or more times in the counting process, the banknote handling apparatus 101 displays, on the display 82, information instructing the operator to move the banknotes from the dispensing unit 12 to the depositing unit 11 as shown in FIG. 11B, after completing the process shown in FIGS. 10A and 10B, in which the banknotes are disinfected twice and discharged to the dispensing unit 12. Upon confirming the instruction on the display 82, the operator moves the banknotes from the dispensing unit 12 to the depositing unit 11, whereby the banknotes are again disinfected. After the banknotes have been disinfected by the designated number of times, the banknote handling apparatus 101 displays, on the display 82, the recognition result including the total amount of the banknotes, thereby completing the counting process. The banknote disinfection can be performed an odd number of times or an even number of times. For example, when disinfecting the banknotes three times, the banknotes may be disinfected twice in the process illustrated in FIGS. 10A and 10B and once in the process illustrated in FIG. 9. When disinfecting the banknotes four times, the banknotes may be disinfected four times by repeating the process illustrated in FIGS. 10A and 10B twice. Similarly, in the disinfection process in the second mode using the temporary storage unit 50, the operator moves the banknotes from the dispensing unit 12 to the depositing unit 11, whereby the banknotes can be disinfected a plural number of times.

In the counting process in which the banknotes are moved from the dispensing unit 12 to the depositing unit 11 in order to repeat the banknote disinfection, the banknotes may not necessarily be recognized each time the banknotes are fed out from the depositing unit 11 into the apparatus. For example, the banknotes may be recognized only once in the first process or the last process of feeding out the banknotes from the depositing unit 11 into the apparatus.

The banknote handling apparatus 101 can perform a money exchange process. In the money exchange process, an amount of money equivalent to the total amount of one or a plurality of banknotes deposited from the depositing unit 11 is dispensed by using one or a plurality of banknotes which are fed out from the storage unit 30 and different from the deposited banknotes.

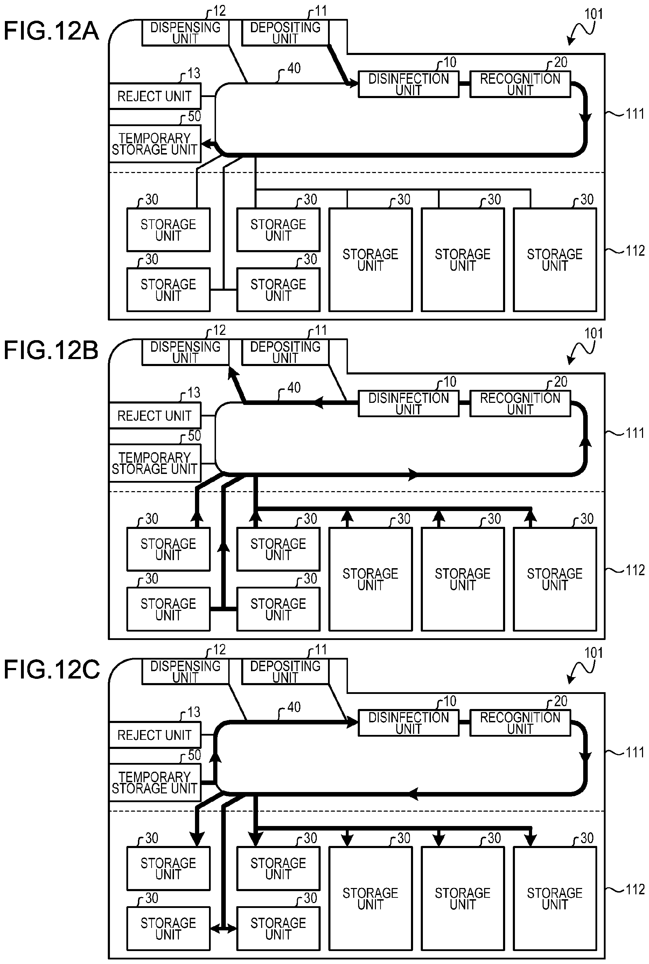

FIGS. 12A to 12C illustrate the money exchange process in the first mode. The operator puts, in the depositing unit 11, banknotes to be exchanged, and designates the denomination and/or the number of banknotes after exchange, by operating the operation unit 81, thereby starting the money exchange process.

As shown in FIG. 12A, the banknote handling apparatus 101 feeds out the banknotes from the depositing unit 11 to the transport unit 40 one by one, disinfects each banknote by the disinfection unit 10, recognizes each banknote by the recognition unit 20, and thereafter stores each banknote in the temporary storage unit 50 temporarily.

As shown in FIG. 12B, based on the designation performed by the operator in advance, the banknote handling apparatus 101 feeds out banknotes, of which the amount is equivalent to the total amount of the temporarily stored banknotes, from one or a plurality of storage units 30 one by one. Each banknote fed out from the storage unit 30 is transported to and recognized by the recognition unit 20, and discharged to the dispensing unit 12. The exchanged banknotes are stacked in the dispensing unit 12. The operator can take out the exchanged banknotes from the dispensing unit 12 to finish the money exchange process. As shown in FIG. 12C, the banknote handling apparatus 101 feeds out the banknotes temporarily stored in the temporary storage unit 50 to the transport unit 40 one by one, disinfects each banknote by the disinfection unit 10, recognizes each banknote by the recognition unit 20, and stores each banknote in the storage unit 30 based on the recognition result, thereby completing the money exchange process.

The money exchange process is not limited to the method illustrated in FIGS. 12A to 12C. The banknote handling apparatus 101 may store the banknotes before exchange, to the storage unit 30 by performing the depositing process illustrated in FIG. 3 or FIGS. 4A and 4B, and thereafter may dispense the exchanged banknotes from the storage unit 30 by performing the dispensing process illustrated in FIG. 5 or FIGS. 6A and 6B.

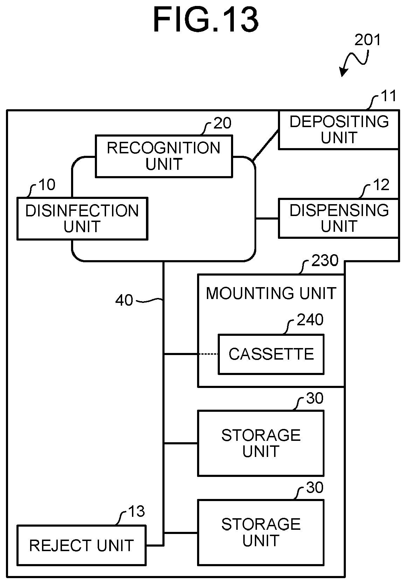

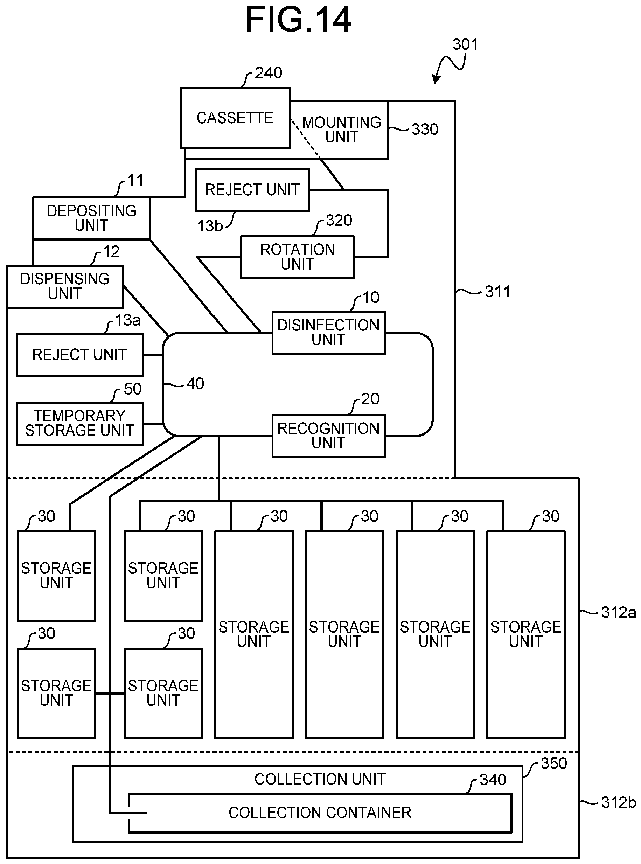

Next, an example of a banknote handling apparatus including a cassette type banknote storage unit (hereinafter referred to as "cassette"), which can be mounted to and dismounted from the apparatus, will be described. FIG. 13 shows an example of a banknote handling apparatus 201 to which a cassette 240 can be dismountably mounted. FIG. 14 shows an example of another banknote handling apparatus 301 to which the same cassette 240 can be dismountably mounted.

Although installation places of the banknote handling apparatuses 201, 301 are not particularly limited, the description will be continued with an example in which the banknote handling apparatus 201 is installed at a checkout counter of a retail store while the banknote handling apparatus 301 is installed in a back office of the retail store.

The banknote handling apparatus 201 shown in FIG. 13 is used for checkout when transaction with a customer is performed at the checkout counter. The banknote handling apparatus 201 can perform depositing of money received from the customer, and dispensing of change to be returned to the customer. The banknote handling apparatus 301 shown in FIG. 14 is used in the back office for managing banknotes in the store. The banknote handling apparatus 301 can perform depositing of banknotes collected from the banknote handling apparatus 201, and dispensing of banknotes with which the banknote handling apparatus 201 is to be replenished. Hereinafter, for simplification, the banknote handling apparatus 201 on the checkout counter is referred to as "front apparatus 201", and the banknote handling apparatus 301 in the back office is referred to as "back apparatus 301".

The front apparatus 201 shown in FIG. 13 includes a reject unit 13 and a mounting unit 230 to which the cassette 240 can be dismountably mounted, in addition to the components shown in FIG. 1. For example, a reject banknote, which is determined not to be deposited based on the recognition result of the recognition unit 20 among banknotes put in the depositing unit 11 for the depositing process, is stored in the reject unit 13. The reject banknote is taken out from the reject unit 13 and checked later.

The cassette 240 can be mounted to and dismounted from the mounting unit 230. When the cassette 240 is mounted to the mounting unit 230, the front apparatus 201 can feed out banknotes stored in the cassette 240 to the transport unit 40. Moreover, the front apparatus 201 can store the banknotes transported by the transport unit 40 into the cassette 240.

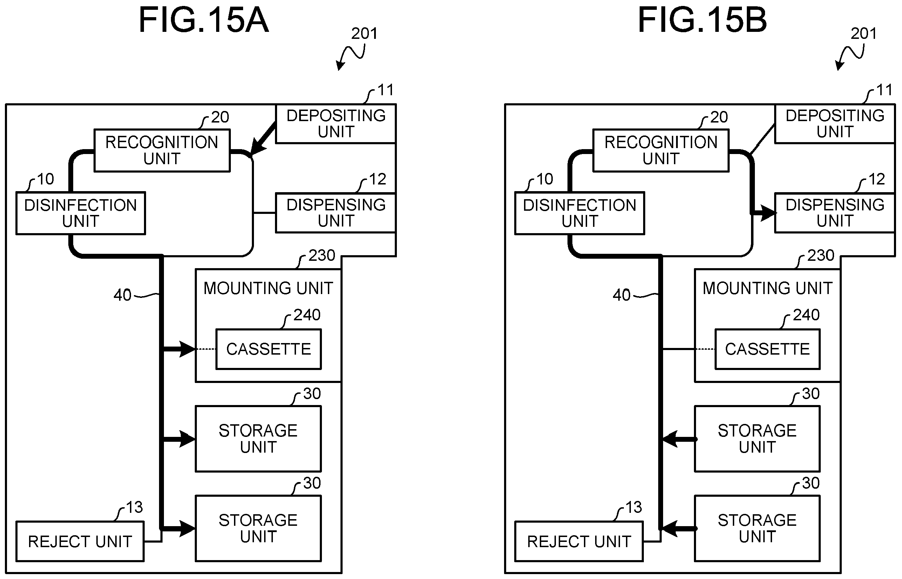

FIGS. 15A and 15B illustrate the depositing process in the first mode and the dispensing process in the first mode, respectively, of the front apparatus 201. In the depositing process, as shown in FIG. 15A, a plurality of banknotes received in the depositing unit 11 are fed out one by one to the transport unit 40 inside the apparatus, recognized by the recognition unit 20, disinfected by the disinfection unit 10, and transported to any of the mounting unit 230 and the plurality of storage units 30. For example, a banknote of which the denomination is assigned to a storage unit 30 is stored in the storage unit 30, and a banknote of which the denomination is assigned to none of the storage units 30 is transported to the mounting unit 230 and stored in the cassette 240.

FIGS. 15A and 15B each show two storage units 30. Two drums, each for storing banknotes while reeling up the banknotes together with a tape onto an outer circumferential surface thereof, are disposed in each storage unit 30, so that two denominations of banknotes can be separately stored in each storage unit 30. The storage unit 30 can feed out the stored banknotes to the transport unit 40 by rotating each drum in a direction opposite to the direction for storing banknotes. That is, the front apparatus 201 uses the two storage units 30 to store banknotes of four denominations by denomination and feed out the stored banknotes to the transport unit 40.

In the dispensing process, banknotes to be dispensed are fed out from the storage unit 30 to the transport unit 40 as shown in FIG. 15B. The fed out banknotes are disinfected by the disinfection unit 10 and recognized by the recognition unit 20, and thereafter these banknotes are dispensed from the dispensing unit 12.

The back apparatus 301 shown in FIG. 14 includes, in addition to the components shown in FIG. 1, two reject units 13 (13a, 13b), a rotation unit 320, a mounting unit 330 to which a cassette 240 can be dismountably mounted, and a collection unit 350 that stores banknotes in a collection container 340.

The back apparatus 301 can store reject banknotes in the two reject units 13a, 13b. For example, the back apparatus 301 can store, in the reject unit 13a, a reject banknote that is determined not to be deposited in the depositing process, and store, in the reject unit 13b, a reject banknote that is determined not to be dispensed in the dispensing process.

The rotation unit 320 can rotate the top/bottom orientation of each banknote without changing the face/back orientation of the banknote. For example, the rotation unit 320 includes a plurality of rollers arranged in a direction orthogonal to the banknote transport direction. These rollers come into contact with the banknote surface and rotate at different rotation speeds, whereby the orientation of the banknote is changed. The banknotes in the cassette 240 mounted to the mounting unit 330 are fed out such that the long edge of each banknote is parallel to the transport direction. Meanwhile, the banknotes in the depositing unit 11 are fed out such that the short edge of each banknote is parallel to the transport direction. The rotation unit 320 rotates each banknote fed out from the cassette 240 by 90 degrees such that the short edge of the banknote becomes parallel to the transport direction, whereby the back apparatus 301 can handle this banknote similarly to the banknote fed out from the depositing unit 11.

The cassette 240 can be mounted to and dismounted from the mounting unit 330. When the cassette 240 is mounted to the mounting unit 330, the back apparatus 301 can feed out the banknotes stored in the cassette 240 to the transport unit 40. Moreover, the back apparatus 301 can store the banknotes transported by the transport unit 40, into the cassette 240.

The collection unit 350 receives the banknotes transported by the transport unit 40, and stores the banknotes in the collection container 340. For example, a bag capable of containing a large number of banknotes is used as the collection container 340. While the cassette 240 and the storage unit 30 perform storage and feeding-out of banknotes, the collection unit 350 performs only storage of banknotes into the collection container 340 and does not feed out banknotes from the collection container 340.

The back apparatus 301 is divided into an upper unit 311 and a lower unit 312. The lower unit 312 is a safe. The lower unit 312 is divided into a first safe unit 312a and a second safe unit 312b. A plurality of storage units 30 for storing a large number of banknotes are disposed in the first safe unit 312a, and the collection container 340 and the collection unit 350 are disposed in the second safe unit 312b.

The transport unit 40 can transport banknotes between the upper unit 311, and the first safe unit 312a and the second safe unit 312b in the lower unit 312. For example, a denomination of banknotes to be stored in each storage unit 30 is set in advance. For example, in the depositing process, deposited banknotes are recognized by the recognition unit 20 in the upper unit 311, transported into the safe, and stored by denomination in the plurality of storage units 30 in the first safe unit 312a. For example, in the dispensing process, banknotes fed out from the storage units 30 are transported from the first safe unit 312a to the upper unit 311, and dispensed from the dispensing unit 12. When collecting the banknotes in the storage unit 30 from the back apparatus 301, the banknotes to be collected are fed out from the storage unit 30, transported from the first safe unit 312a to the collection unit 350 in the second safe unit 312b, and stored in the collection container 340 by the collection unit 350. The operator can open a safe door of the lower unit 312, and removes, from the collection unit 350, the collection container 340 in which the banknotes to be collected are being stored, thereby collecting the banknotes to outside the back apparatus 301.

Like the banknote handling apparatus 101 shown in FIG. 2, the back apparatus 301 can perform banknote handling processes such as a depositing process, a dispensing process, an inventory confirming process, an intra-apparatus disinfection process, a counting process, a disinfection process, and a money exchange process. Since these banknote handling processes in the back apparatus 301 are performed in the same way as the banknote handling processes performed in the banknote handling apparatus 101 shown in FIG. 2 as illustrated in FIG. 3 to FIG. 12C, the detailed description thereof is omitted.

The back apparatus 301 can perform a depositing process for banknotes in the cassette 240. The back apparatus 301 can perform the depositing process not using the temporary storage unit 50, and/or the depositing process using the temporary storage unit 50. The operator can change the type of the depositing process to be performed in the back apparatus 301.

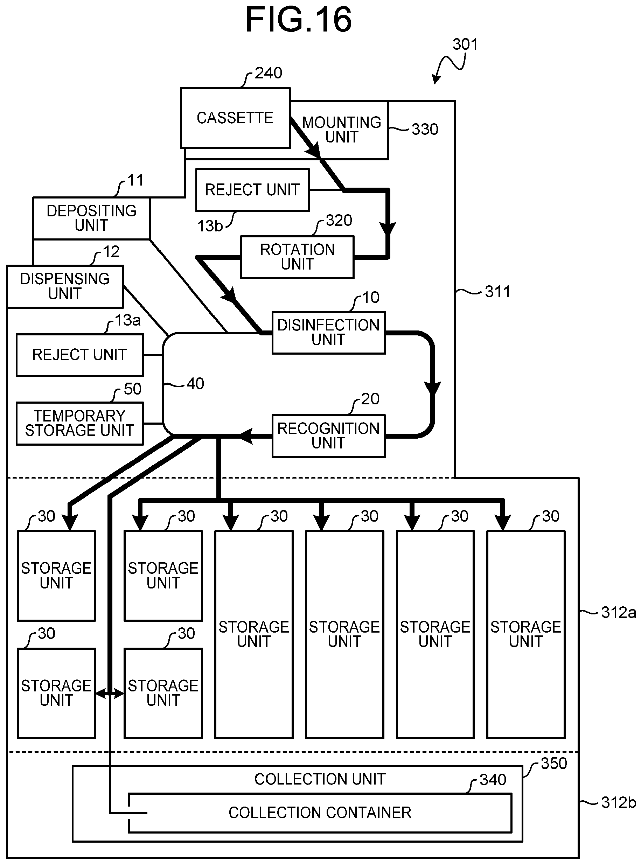

FIG. 16 illustrates the depositing process for the banknotes in the cassette 240 performed without using the temporary storage unit 50. This depositing process is performed in the first mode. Banknotes are fed out from the cassette 240 mounted to the mounting unit 330, to the transport unit 40 one by one. The transport unit 40 transports each banknote fed out from the cassette 240, to the rotation unit 320. Each banknote is transported, from the mounting unit 330 to the rotation unit 320, with the long edge thereof is parallel to the transport direction. The rotation unit 320 rotates the top/bottom orientation of each banknote by 90 degrees without changing the face/back orientation of each banknote such that the short edge of each banknote becomes parallel to the transport direction. Thus, after each banknote has been transported from the rotation unit 320 to the disinfection unit 10, the back apparatus 301 can handle each banknote similarly to the banknote deposited from the depositing unit 11. The disinfection unit 10 disinfects each banknote transported from the rotation unit 320. Thereafter, each banknote is recognized by the recognition unit 20, and transported to and stored in any of the storage units 30 corresponding to the recognition result.

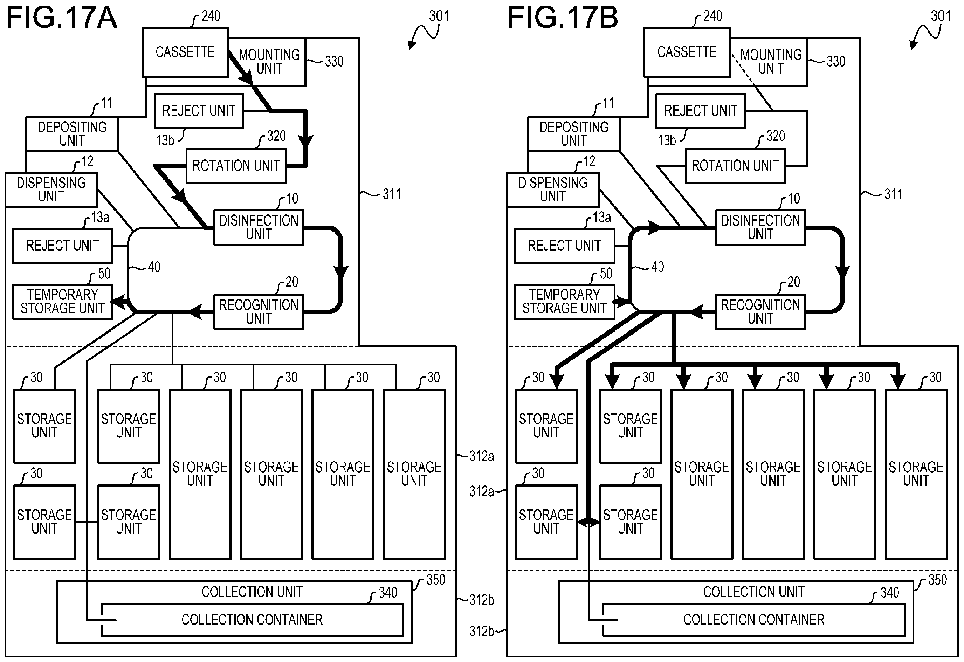

FIGS. 17A and 17B illustrate the depositing process for the banknotes in the cassette 240 performed by using the temporary storage unit 50. This depositing process is performed in the first mode. The depositing process using the temporary storage unit 50 includes: a temporary storage process of feeding out the banknotes from the cassette 240 and temporarily storing the banknotes in the temporary storage unit 50; and a storage process of feeding out the banknotes from the temporary storage unit 50 and storing the banknotes in the storage unit 30.

FIG. 17A shows movement of the banknotes in the temporary storage process. Banknotes are fed out from the cassette 240 mounted to the mounting unit 330 to the transport unit 40 one by one. The transport unit 40 transports each banknote fed out from the cassette 240, to the rotation unit 320. The rotation unit 320 rotates the top/bottom orientation of each banknote by 90 degrees without changing the face/back orientation of each banknote such that the short edge of each banknote becomes parallel to the transport direction. Then, each banknote is disinfected by the disinfection unit 10 and recognized by the recognition unit 20, and thereafter each banknote is temporarily stored in the temporary storage unit 50. After all the banknotes to be deposited from the cassette 240 have been temporarily stored in the temporary storage unit 50, the storage process of storing these banknotes into the storage units 30 is started.

After the temporary storage process has been completed, the recognition result of the banknotes temporarily stored in the temporary storage unit 50 is displayed on the display 82. The recognition result includes the total amount of the banknotes temporarily stored in the temporary storage unit 50, that is, the amount of banknotes to be deposited. Upon confirming the information on the display 82, the operator operates the operation unit 81 to approve the recognition result and finish the depositing process. Then, the storage process is started.

FIG. 17B shows movement of the banknotes in the storage process. The temporary storage unit 50 feeds out all the temporarily stored banknotes to the transport unit 40 one by one. The transport unit 40 transports each banknote from the temporary storage unit 50 to the disinfection unit 10. The disinfection unit 10 disinfects each banknote being transported by the transport unit 40. Thereafter, each banknote is recognized by the recognition unit 20, transported to the first safe unit 312a in the lower unit 312, and stored in any of the storage units 30 corresponding to the recognition result. Thus, all the banknotes temporarily stored in the temporary storage unit 50, that is, all the deposited banknotes, are stored in the storage units 30.

In the above example, the recognition unit 20 recognizes the banknotes in both the temporary storage process of transporting the banknotes from the cassette 240 to the temporary storage unit 50 in order to store the banknotes therein, and the storage process of transporting the banknotes from the temporary storage unit 50 to the storage unit 30 in order to store the banknotes therein. However, the recognition unit 20 may recognize the banknotes only once in the temporary storage process or the storage process.

The back apparatus 301 can perform a dispensing process of storing the dispensed banknotes into the cassette 240. The back apparatus 301 can perform the dispensing process not using the temporary storage unit 50, and/or the dispensing process using the temporary storage unit 50. The operator can change the type of the dispensing process to be performed in the back apparatus 301. For example, the operator of the back apparatus 301 operates the operation unit 81 to start the dispensing process and designate banknotes to be dispensed. Alternatively, for example, information designating banknotes to be dispensed is inputted from the external device to the back apparatus 301 through the communication unit 90.

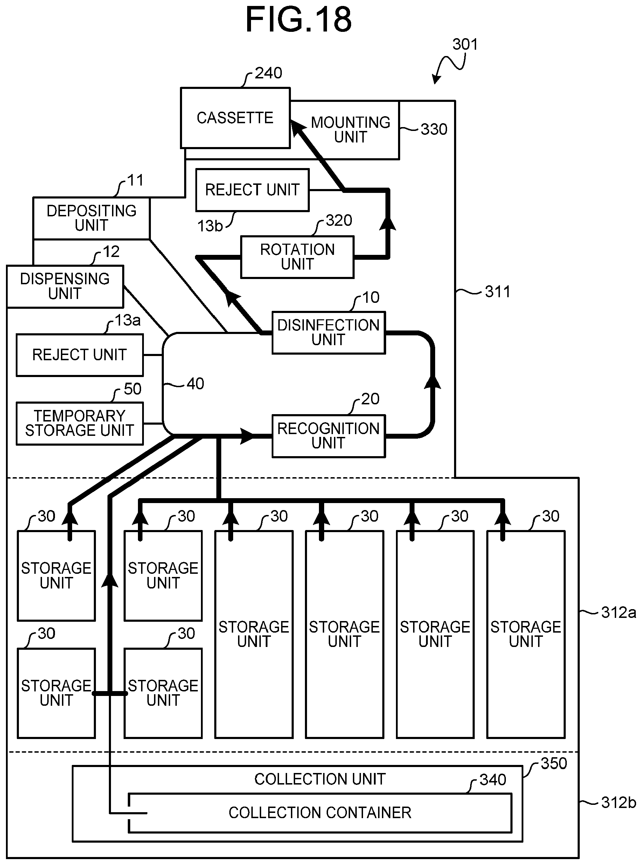

FIG. 18 illustrates the dispensing process to the cassette 240 performed without using the temporary storage unit 50. This dispensing process is performed in the first mode. Banknotes to be dispensed are fed out from one or a plurality of storage units 30 to the transport unit 40 one by one. The transport unit 40 transports each banknote fed out from the storage unit 30 to the recognition unit 20 one by one. Each banknote is recognized by the recognition unit 20 and disinfected by the disinfection unit 10, and thereafter each banknote is transported to the rotation unit 320. Each banknote is transported, from the storage unit 30 to the rotation unit 320, with the short edge thereof is parallel to the transport direction. On the other hand, the cassette 240 stores a banknote transported with the long edge thereof is parallel to the transport direction. Therefore, the rotation unit 320 rotates the top/bottom orientation of each banknote by 90 degrees without changing the face/back orientation of each banknote such that the long edge of each banknote becomes parallel to the transport direction. Thus, the cassette 240 can store each banknote transported from the rotation unit 320 to the mounting unit 330. Thus, all the banknotes to be dispensed are stored in the cassette 240 to complete the dispensing process.

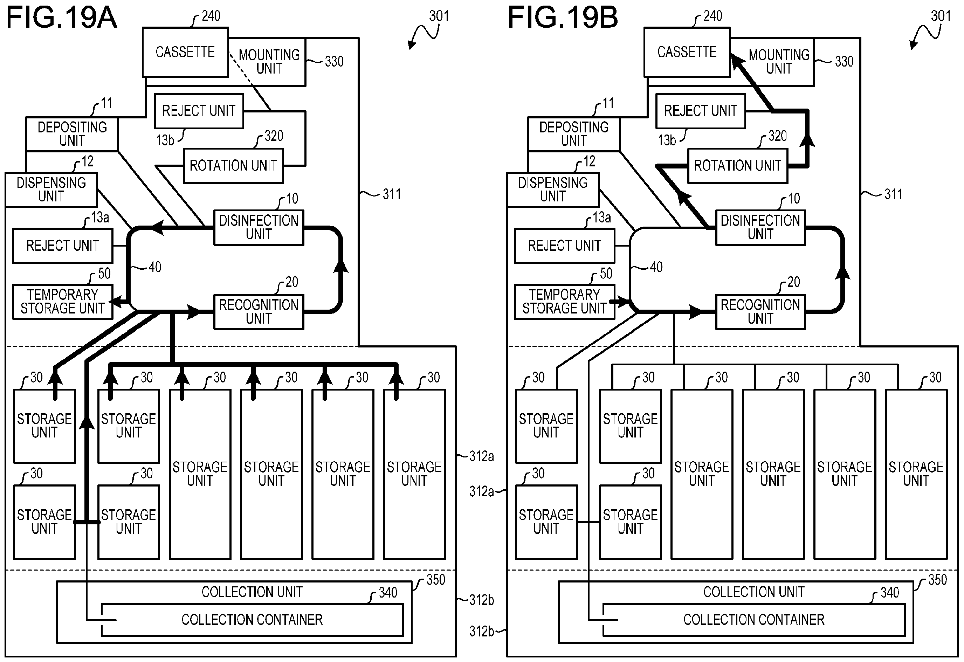

FIGS. 19A and 19B illustrate a dispensing process to the cassette 240 performed by using the temporary storage unit 50. This dispensing process is performed in the first mode. The dispensing process using the temporary storage unit 50 includes: a temporary storage process of feeding out banknotes from the storage unit 30 and temporarily storing the banknotes in the temporary storage unit 50; and a storage process of feeding out the banknotes from the temporary storage unit 50 and storing the banknotes in the cassette 240.

FIG. 19A shows movement of the banknotes in the temporary storage process. The banknotes to be dispensed are fed out from one or a plurality of storage units 30 to the transport unit 40 one by one. The transport unit 40 transports the fed out banknotes to the recognition unit 20 one by one. Each banknote is recognized by the recognition unit 20, disinfected by the disinfection unit 10 and stored in the temporary storage unit 50 temporarily. After all the banknotes to be dispensed have been temporarily stored in the temporary storage unit 50, the storage process of storing these banknotes into the cassette 240 is started.

FIG. 19B shows movement of the banknotes in the storage process. The temporary storage unit 50 feeds out all the temporarily stored banknotes to the transport unit 40 one by one. The transport unit 40 transports each banknote to the recognition unit 20. Each banknote is recognized by the recognition unit 20 and disinfected by the disinfection unit 10, and thereafter each banknote is transported to the rotation unit 320. The rotation unit 320 rotates the top/bottom orientation of each banknote by 90 degrees without changing the face/back orientation of each banknote such that the long edge of each banknote becomes parallel to the transport direction. Each banknote is transported from the rotation unit 320 to the mounting unit 330 and stored in the cassette 240.

In the above example, the recognition unit 20 recognizes the banknotes in both the temporary storage process of transporting the banknotes from the storage unit 30 to the temporary storage unit 50 in order to store the banknotes therein, and the storage process of transporting the banknotes from the temporary storage unit 50 to the cassette 240 in order to store the banknotes therein. However, the recognition unit 20 may recognize the banknotes only once in the temporary storage process or the storage process.

The banknotes deposited in the front apparatus 201 are disinfected once by the disinfection unit 10 and thereafter the banknotes are stored in the storage unit 30, as shown in FIG. 15A. When collecting the banknotes from the front apparatus 201 and depositing the collected banknotes into the back apparatus 301 by using the cassette 240, the banknotes to be collected are fed out from the storage unit 30 in the front apparatus 201 and stored in the cassette 240. The cassette 240 in which the banknotes to be collected are stored is removed from the mounting unit 230 of the front apparatus 201, and mounted to the mounting unit 330 of the back apparatus 301.

In the case where the back apparatus 301 performs the depositing process without using the temporary storage unit 50 as shown in FIG. 16, each banknote fed out from the cassette 240 is disinfected once and stored in the storage unit 30. As a result, the banknote is disinfected twice between the time of depositing in the front apparatus 201 and the time of storing in the storage unit 30 of the back apparatus 301. On the other hand, in the case where the back apparatus 301 performs the depositing process by using the temporary storage unit 50 as shown in FIGS. 17A and 17B, each banknote fed out from the cassette 240 is disinfected twice inside the back apparatus 301. Therefore, the banknote is disinfected three times between the time of depositing in the front apparatus 201 and the time of storing in the storage unit 30 of the back apparatus 301.

When the banknotes stored in the storage unit 30 of the back apparatus 301 are dispensed by using the cassette 240, the banknotes are further disinfected. In the case where the back apparatus 301 performs the dispensing process without using the temporary storage unit 50 as shown in FIG. 18, each banknote in the storage unit 30 is disinfected once and thereafter stored in the cassette 240. As a result, the banknote is disinfected three or four times between the time of depositing in the front apparatus 201 and the time of storing in the cassette 240 of the back apparatus 301. On the other hand, in the case where the back apparatus 301 performs the dispensing process by using the temporary storage unit 50 as shown in FIGS. 19A and 19B, each banknote fed out from the storage unit 30 is disinfected twice inside the back apparatus 301. Therefore, the banknote is disinfected four or five times between the time of depositing in the front apparatus 201 and the time of storing in the cassette 240 of the back apparatus 301. For example, in the case where the cassette 240 removed from the back apparatus 301 is carried from the store to a financial institution and mounted to a banknote handling apparatus of the same model as the back apparatus 301 to perform a depositing process, the banknotes in the cassette 240 are further disinfected.

As described above, since the banknotes are moved by using the cassette 240 between the plurality of banknote handling apparatuses each having the disinfection unit 10, the banknotes can be disinfected a plural number of times while the banknotes are stored in the cassette 240 or in the banknote handling apparatuses without being touched by operator's hands and customer's hands.

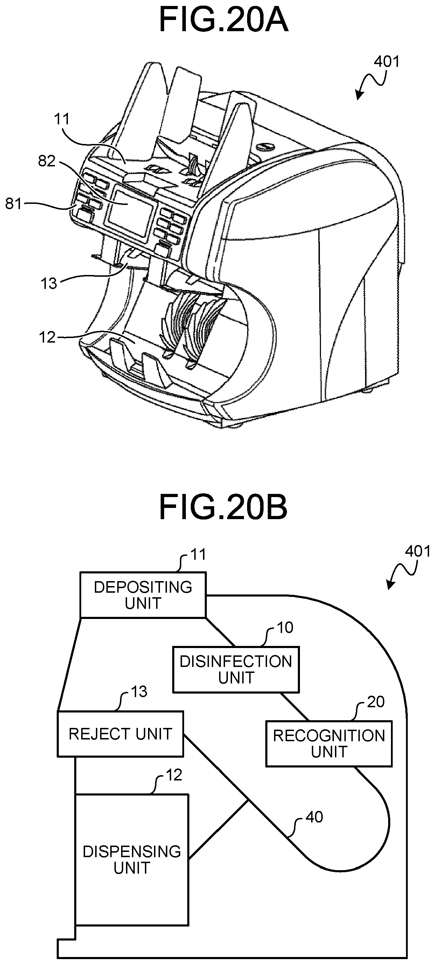

Next, an example of a banknote handling apparatus in which banknotes are not stored in a storage unit 30 will be described. FIGS. 20A and 20B illustrate an example of a banknote handling apparatus 401 that does not store banknotes inside the apparatus. FIG. 20A shows an external appearance of the banknote handling apparatus 401, and FIG. 20B shows a schematic internal configuration of the banknote handling apparatus 401.

The banknote handling apparatus 401 performs a counting process and a disinfection process for banknotes. The banknote handling apparatus 401 includes an operation unit 81, a display 82 and a depositing unit 11 on which a plurality of banknotes can be placed. The depositing unit 11 is provided at an upper portion of the apparatus 401. The operation unit 81 and the display 82 are provided at a front face of the apparatus 401, beneath the depositing unit 11.

In the counting process in the first mode, banknotes placed on the depositing unit 11 are fed out to the transport unit 40 inside the apparatus 401 one by one. Each banknote being transported by the transport unit 40 is disinfected by the disinfection unit 10 and recognized by the recognition unit 20, and thereafter each banknote is discharged to the reject unit 13 or the dispensing unit 12 based on the recognition result. The recognition result of the banknotes is displayed on the display 82. For example, at least one of the denominations of banknotes stacked in the dispensing unit 12, the number of banknotes for each denomination, the total number of the banknotes, and the total amount of the banknotes, is displayed on the display 82. In addition, information on reject banknotes stacked in the reject unit 13 may be displayed on the display 82. The disinfection process in the second mode by the banknote handling apparatus 401 can be performed in a similar manner to the counting process in the first mode except that recognition by the recognition unit 20 is not performed.

The banknote handling apparatus 401 can disinfect the same banknote a plural number of times. The number of times of the banknote disinfection can be set in advance, or can be designated by the operator. For example, when disinfecting the banknotes twice in the counting process, the banknote handling apparatus 401 displays, on the display 82, information indicating that the banknotes are being subjected to the first disinfection, similar to the screen shown in FIG. 11A, after starting the feeding-out of the banknotes from the depositing unit 11.

After all the banknotes in the depositing unit 11 have been fed out into the apparatus, disinfected, recognized and stacked in the dispensing unit 12 or the reject unit 13, the banknote handling apparatus 401 displays, on the display 82, information instructing the operator to take out the banknotes from the dispensing unit 12 and the reject unit 13 and place these banknotes on the depositing unit 11, similar to the screen shown in FIG. 11B.

After the operator has moved the banknotes from the dispensing unit 12 and the reject unit 13 to the depositing unit 11, the banknotes on the depositing unit 11 are fed out into the apparatus again, and subjected to the second disinfection and recognition. For example, when the operator performs a predetermined operation on the operation unit 81 after placing the banknotes on the depositing unit 11, the second handling process is started. Alternatively, for example, a sensor disposed in the depositing unit 11 detects the banknotes placed on the depositing unit 11 by the operator, thereby starting the second handling process.

The banknote handling apparatus 401, having started the second disinfection and recognition, displays, on the display 82, a screen similar to the screen shown in FIG. 11A to notify the operator that the second banknote disinfection is being performed. After all the banknotes in the depositing unit 11 have been disinfected and recognized, the banknote handling apparatus 401 displays a banknote recognition result on the display 82. Likewise, when banknote disinfection is to be performed three or more times, disinfection of banknotes received on the depositing unit 11, stacking of the banknotes in the dispensing unit 12 or the reject unit 13, and movement of the banknotes from the dispensing unit 12 and the reject unit 13 to the depositing unit 11, are repeated. When the banknote handling apparatus 401 performs the disinfection process a plural number of times, the banknotes are handled in a similar manner to the counting process except that the banknote recognition by the recognition unit 20 is not performed.

Since the banknote handling apparatus 401 is a small desktop-type apparatus, the ultraviolet ray emitted from the disinfection unit 10 may be more likely to leak to the outside of the apparatus through a gap formed in the depositing unit 11, the reject unit 13, the dispensing unit 12, and the like, as compared to a large banknote handling apparatus. Therefore, the disinfection unit 10 preferably uses a light source emitting an ultraviolet ray that does not adversely affect the human body, such as a light source emitting an ultraviolet ray at a wavelength of 222 nm.

Although the disinfection unit 10 is disposed between the depositing unit 11 and the recognition unit 20 in the example shown in FIG. 20B, the disinfection unit 10 may be disposed downstream of the recognition unit 20 in the transport direction. A plurality of disinfection units 10 may be disposed inside the apparatus 401. For example, disinfection units 10 may be disposed on both the upstream side and the downstream side of the recognition unit 20 in the transport direction.

In the present embodiment, the disinfection unit 10 irradiates a banknote with an ultraviolet ray to disinfect the banknote. However, the banknote disinfection method is not limited thereto. An appropriate disinfection method may be determined according to the type of a pathogen to be disinfected.

For example, the disinfection unit 10 may irradiate banknotes with a ray other than an ultraviolet ray, or radiation. The disinfection unit 10 may disinfect banknotes by an alternative method such as heating, or spraying of disinfectant. The disinfection unit 10 may not necessarily perform only one type of disinfection, and may perform two or more types of disinfection. For example, the disinfection unit 10 may irradiate banknotes with two types of rays in different wavelength ranges. The disinfection unit 10 may perform two or more types disinfection selected from among irradiation with light, irradiation with radiation, heating, spraying of disinfectant, and the like.

In the present embodiment, the disinfection unit 10 is disposed along the transport unit 40 and disinfects the banknotes being transported by the transport unit 40. However, the banknote disinfection method is not limited thereto. For example, when the disinfection unit 10 performs disinfection, the transport unit 40 may stop transport of banknotes. For another example, after the disinfection unit 10 has received a banknote from the transport unit 40, taken the banknote inside the disinfection unit 10, and disinfected the banknote, the disinfection unit 10 may feed out the banknote to the transport unit 40.

As described above, the banknote handling apparatus according to the present embodiment can disinfect banknotes inside the banknote handling apparatus. The banknote handling apparatus can disinfect the banknotes a plural number of times during one banknote handling process. The banknote handling apparatus can disinfect the banknotes a plural number of times while the banknotes are stored in a storage unit inside the apparatus. After receiving banknotes from outside the apparatus and disinfecting the received banknotes, the banknote handling apparatus can discharge the banknotes without storing these banknotes inside the apparatus. When performing a banknote handling process, the banknote handling apparatus can set the process so as to perform banknote disinfection or so as not to perform banknote disinfection. By selecting and performing either the banknote handling process with banknote disinfection or the same banknote handling process without banknote disinfection according to need, it is possible to efficiently perform banknote handling and banknote disinfection.

As described above, the banknote handling apparatus and the banknote handling method according to the present disclosure are useful in efficiently performing banknote handling and banknote disinfection.

## Claims

1. A banknote handling apparatus (1), comprising:
an inlet (11) configured to receive a banknote from outside the banknote handling apparatus (1);
a transport unit (40) configured to transport the banknote fed out from the inlet (11) into the banknote handling apparatus (1);
a recognition unit (20) configured to perform banknote recognition to recognize the banknote being transported by the transport unit (40);
a disinfection unit (10) configured to perform banknote disinfection to disinfect the banknote being transported by the transport unit (40);
an outlet (12) configured to hold the banknote received from the transport unit (40) such that the banknote can be taken out from the banknote handling apparatus (1); and
a control unit (60) configured to control the transport unit (40), the recognition unit (20), and the disinfection unit (10) to perform a banknote handling process in which the banknote being transported by the transport unit (40) passes the recognition unit (20) and the disinfection unit (10), wherein
the banknote handling apparatus (1) performs the banknote handling process in a mode selected from among a plurality of modes, and
the plurality of modes includes a first mode in which the banknote recognition and the banknote disinfection are performed, a second mode in which the banknote disinfection is performed without performing the banknote recognition, and a third mode in which the banknote recognition is performed without performing the banknote disinfection.

2. The banknote handling apparatus (1) according to claim 1, wherein the transport unit (40) transports the banknote at a predetermined transport speed, and the transport speed in the first mode is different from the transport speed in the third mode.

3. The banknote handling apparatus (1) according to claim 1 or 2, wherein the transport unit (40) transports the banknote at a predetermined transport speed, and the transport speed in the second mode is different from the transport speed in the third mode.

4. The banknote handling apparatus (1) according to any one of claims 1 to 3, wherein the disinfection unit (10) disinfects the same banknote a plural number of times during the banknote handling process in the first mode or the second mode.

5. The banknote handling apparatus (1) according to claim 4, wherein in a case where the disinfection unit (10) disinfects the banknote a plural number of times during the banknote handling process, the transport unit (40) changes a transport speed of the banknote such that the transport speed of a first banknote disinfection is different from the transport speed of a second banknote disinfection.

6. The banknote handling apparatus (1) according to claim 4 or 5, further comprising a display that displays information indicating a number of times of the banknote disinfection in the case where the disinfection unit (10) disinfects the same banknote a plural number of times.

7. The banknote handling apparatus (1) according to any one of claims 1 to 6, wherein
the recognition unit (20) is configured to recognize a number of banknotes, denomination, authenticity, and fitness of each banknote, and
the control unit (60) controls the recognition unit (20) not to recognize at least one of the number of banknotes, the denomination, the authenticity, and the fitness in a predetermined banknote handling process in the first mode or the third mode.

8. The banknote handling apparatus (1) according to any one of claims 1 to 7, wherein in the banknote handling process, the inlet (11) feeds out the banknote received from outside the banknote handling apparatus (1), the transport unit (40) transports the banknote fed out from the inlet (11), and the outlet (12) receives the banknote transported by the transport unit (40).

9. A banknote handling apparatus (1) according to any one of claims 1 to 7, further comprising:
a storage unit (30) configured to store the banknote received from the transport unit (40), and feed out the stored banknote to the transport unit (40); and
a temporary storage unit (50) configured to temporarily store the banknote received from the transport unit (40), and feed out the temporarily stored banknote to the transport unit (40), wherein
in the banknote handling process, the transport unit (40) transports the banknote fed out from the storage unit (30), and the temporary storage unit (50) receives the banknote transported by the transport unit (40) and temporarily stores the banknotes therein.

10. A banknote handling apparatus (1) according to any one of claims 1 to 7, further comprising:
a storage unit (30) configured to store the banknotes received from the transport unit (40), and feed out the stored banknotes to the transport unit (40); and
a temporary storage unit (50) configured to temporarily store the banknotes received from the transport unit (40), and feed out the temporarily stored banknotes to the transport unit (40), wherein
in the banknote handling process, the transport unit (40) transports the banknotes fed out from the temporary storage unit (50), and the storage unit (30) receives the banknotes transported by the transport unit (40) and stores the banknotes therein.

11. A banknote handling apparatus (1) according to any one of claims 1 to 7, further comprising:
a storage unit (30) configured to store the banknotes received from the transport unit (40), and feed out the stored banknotes to the transport unit (40); and
a temporary storage unit (50) configured to temporarily store the banknotes received from the transport unit (40), and feed out the temporarily stored banknotes to the transport unit (40), wherein
in the banknote handling, the transport unit (40) transports the banknotes fed out from the storage unit (30), the temporary storage unit (50) receives the banknotes transported by the transport unit (40) and temporarily stores the banknotes therein, the transport unit (40) transports the banknotes fed out from the temporary storage unit (50), and the storage unit (30) receives the banknotes transported by the transport unit (40) and stores the banknotes therein.

12. The banknote handling apparatus (1) according to any one of claims 1 to 11, wherein the recognition unit (20) and the disinfection unit (10) are disposed adjacent to each other on the transport unit (40).

13. The banknote handling apparatus (1) according to any one of claims 1 to 12, wherein the recognition unit (20) and the disinfection unit (10) are connected to each other by a transport path, which has no divergent path and constitutes a part of the transport unit (40).

14. A banknote handling method performed by a banknote handling apparatus (1), the method comprising performing a step selected from among the steps of:
recognizing, by a recognition unit (20), banknotes received from outside the banknote handling apparatus (1), and disinfecting the banknotes by a disinfection unit (10);
disinfecting, by the disinfection unit (10), the banknotes received from outside of the banknote handling apparatus (1), without recognizing the banknotes by the recognition unit (20); and
recognizing, by the recognition unit (20), the banknotes received from outside the banknote handling apparatus (1), without disinfecting the banknotes by the disinfection unit (10) .
